(19) Europäisches Patentamt
European Patent Office
Office européen des brevets

(11) **EP 2 933 874 B1**

(12) **EUROPEAN PATENT SPECIFICATION**

(45) Date of publication and mention of the grant of the patent:
**24.02.2021 Bulletin 2021/08**

(21) Application number: **13863260.9**

(22) Date of filing: **10.12.2013**

(51) Int Cl.:
***H01M 14/00*** *(2006.01)*
***C07D 409/14*** *(2006.01)*
***H01L 31/04*** *(2014.01)*
***H01G 9/20*** *(2006.01)*
***C07D 213/79*** *(2006.01)*
***C07F 15/00*** *(2006.01)*
***C09B 57/10*** *(2006.01)*

(86) International application number:
**PCT/JP2013/083045**

(87) International publication number:
**WO 2014/092066 (19.06.2014 Gazette 2014/25)**

# (54) PHOTOELECTRIC CONVERSION ELEMENT

FOTOELEKTRISCHES UMWANDLUNGSELEMENT

ÉLÉMENT DE CONVERSION PHOTOÉLECTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2012 JP 2012273636**

(43) Date of publication of application:
**21.10.2015 Bulletin 2015/43**

(73) Proprietor: **Sharp Kabushiki Kaisha**
**Osaka-shi, Osaka 545-8522 (JP)**

(72) Inventors:
- **FUKUI, Atsushi**
  **Osaka-shi,**
  **Osaka 545-8522 (JP)**
- **KASAHARA, Kei**
  **Osaka-shi,**
  **Osaka 545-8522 (JP)**
- **ELFASSYFIHRY, Mehdi**
  **Osaka-shi,**
  **Osaka 545-8522 (JP)**
- **KOMIYA, Ryoichi**
  **Osaka-shi,**
  **Osaka 545-8522 (JP)**
- **YAMANAKA, Ryohsuke**
  **Osaka-shi,**
  **Osaka 545-8522 (JP)**

(74) Representative: **Müller Hoffmann & Partner**
**Patentanwälte mbB**
**St.-Martin-Strasse 58**
**81541 München (DE)**

(56) References cited:
**JP-A- 2012 146 632**
**JP-A- 2012 195 280**
**JP-A- 2012 199 096**
**JP-A- 2012 243 436**
**JP-A- 2012 243 436**
**US-A1- 2012 073 660**

- **KUSAMA H ET AL: "Influence of pyrazole derivatives in I<->/I3<-> redox electrolyte solution on Ru(II)-dye-sensitized TiO2 solar cell performance", SOLAR ENERGY MATERIALS AND SOLAR CELLS, ELSEVIER SCIENCE PUBLISHERS, AMSTERDAM, NL, vol. 85, no. 3, 24 June 2004 (2004-06-24), pages 333-344, XP027815031, ISSN: 0927-0248 [retrieved on 2005-01-31]**
- **HITOSHI KUSAMA ET AL: "Influence of nitrogen-containing heterocyclic additives in I-/I3- redox electrolytic solution on the performance of Ru-dye-sensitized nanocrystalline TiO2 solar cell", JOURNAL OF PHOTOCHEMISTRY AND PHOTOBIOLOGY, A: CHEMISTRY., vol. 169, no. 2, 3 August 2004 (2004-08-03), pages 169-176, XP055282083, CH ISSN: 1010-6030, DOI: 10.1016/j.jphotochem.2004.06.012**

EP 2 933 874 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KUAN-LIN WU ET AL.: 'Thiocyanate-Free Ru(II) Sensitizers with a 4,4'-Dicarboxyvinyl-2,2'-bipyridine Anchor for Dye-Sensitized Solar Cells' ADVANCED FUNCTIONAL MATERIALS vol. 23, no. 18, 13 May 2013, pages 2285 - 2294, XP001582794
- KUAN-LIN WU ET AL.: 'Development of thiocyanate-free, charge-neutral Ru(II) sensitizers for dye-sensitized solar cells' CHEMICAL COMMUNICATIONS vol. 46, no. 28, 2010, pages 5124 - 5126, XP055257270
- KUSAMA, H. ET AL.: 'Theoretical studies of charge-transfer complexes 12 with pyrazoles, and implications on the dye-sensitized solar cell performance' JOURNAL OF PHOTOCHEMISTRY AND PHOTOBIOLOGY A: CHEMISTRY vol. 187, no. 2-3, 23 March 2007, pages 233 - 241, XP005934356
- KUSAMA, H. ET AL.: 'Influence of pyrazole derivatives in I /I3 redox electrolyte solution on Ru(II)-dye-sensitized Ti02 solar cell performance' SOLAR ENERGY MATERIALS AND SOLAR CELLS vol. 85, no. 3, 31 January 2005, pages 333 - 344, XP025333185

## Description

Technical Field

The present invention relates to a photoelectric conversion element.

Background Art

Recently, as an energy source replacing fossil fuel, a solar cell which is able to convert solar energy into electric energy has received attention. Currently, a solar cell using a crystalline silicon substrate, a thin film silicon solar cell, and the like are in practical use. However, the former solar cell has a disadvantage of high manufacturing cost of the silicon substrate. The latter thin film silicon solar cell has a disadvantage of high manufacturing cost due to using various gases for manufacturing a semiconductor, a complicated device, and the like. In both of the solar cells, efforts for reducing the cost per power generation output have been continued by increasing efficiency in photoelectric conversion, but have not reached a solution for these problems.

As a new type solar cell, a photoelectro-chemical cell to which a photoinduced electron migration of a metal complex, a photosensitizing dye, or the like is applied has been proposed (for example, PTL 1 (Japanese Unexamined Patent Application Publication No. 1-220380)). In the photoelectro-chemical cell, a photoelectric conversion layer formed of a photoelectric conversion material which has an absorption spectrum in a visible light region by adsorbing the photosensitizing dye and an electrolyte material is interposed between two electrodes in which the electrodes are formed on surfaces of glass substrates (such a solar cell may be referred to as a "dye-sensitized photovoltaic cell"). When the photoelectro-chemical cell is irradiated with light from one electrode side of the solar cell, electrons are generated in the photoelectric conversion layer, and the generated electrons migrate to the counter electrode (the other electrode) through an external electric circuit. The migrated electrons are carried by ions in the electrolyte and return to the photoelectric conversion layer. By such a successive migration of the electrons, it is possible to obtain electric energy.

In order to improve photoelectric conversion efficiency of the dye-sensitized photovoltaic cell, a method has been known in which an additive is added to an electrolyte solution. For example, methods are described in PTL 2 (Japanese Unexamined Patent Application Publication No. 2003-331936), PTL 3 (Japanese Unexamined Patent Application Publication No. 2004-47229), and PTL 4 (Japanese Unexamined Patent Application Publication No. 2005-216490), in which mainly in order to improve an open circuit voltage of the solar cell, 2-n-propyl pyridine, an aminopyridine-based compound, and a pyrazole-based compound are added to the electrolyte solution, respectively. In addition, in PTL 5 (Japanese Unexamined Patent Application Publication No. 2006-134615), mainly in order to improve the open circuit voltage of the solar cell, a solar cell containing a nitrogen-containing heterocyclic compound formed of an aromatic ring such as a 5-membered ring or a 6-membered ring which has two or more nitrogen atoms and does not have a substituent is described. In order to suppress volatilization of the electrolyte solution, the molecular weight of the additive to the electrolyte solution may increase. However, when the molecular weight of the additive to the electrolyte solution increases, an increase in the viscosity of the electrolyte solution is induced, and as a result a decrease in photoelectric conversion efficiency of the solar cell is induced. In PTL 5, a photoelectric conversion element is disclosed in which volatilization of the base is suppressed by using a cyclic base having a small molecule size and a high boiling point, and thus photoelectric conversion efficiency of the solar cell is improved and durability thereof is also improved. In PTL 6, a photoelectric conversion device is disclosed in which the dye is a Ru complex having no thiocyanate groups.

Citation List

Patent Literature

PTL 1: Japanese Unexamined Patent Application Publication No. 1-220380
PTL 2: Japanese Unexamined Patent Application Publication No. 2003-331936
PTL 3: Japanese Unexamined Patent Application Publication No. 2004-47229
PTL 4: Japanese Unexamined Patent Application Publication No. 2005-216490
PTL 5: Japanese Unexamined Patent Application Publication No. 2006-134615
PTL 6: US 2012/073660 A1 Kusama H. et al. (Sol. Ener. Mater. and Sol. Cells, 2004, 85, 333 - 344), JP 2012 243436 A, and Kusama H. et al. (J. Photochem. Photobiol. A, 2004, 169, 169-176) describe N719, Z907, or Z991 as dye, wherein each of these dies has two thiocyanate groups with an N-atom bonding to Ru-atom.

## Summary of Invention

### Technical Problem

In the photoelectric conversion element using dye as a photosensitizing material, a huge barrier to practical use thereof is durability, and performance of the element is degraded over time. In particular, a decrease in performance is significant in an acceleration test such as a heat resistance test of JIS Standard C8639. This has been achieved by the subject-matter of the independent claims.

The present invention is made in consideration of the circumstances described above, and is to provide a photoelectric conversion element in which a decrease in performance due to heat is suppressed.

### Solution to Problem

In a photoelectric conversion element of the present invention, a conductive layer, a photoelectric conversion layer, and a counter electrode are disposed, and at least photoelectric conversion layer is filled with an electrolyte. In the photoelectric conversion layer, at least one of a dye formed of a compound having no more than one thiocyanate group and a dye formed of a compound not having a thiocyanate group is adsorbed ont a porous semiconductor layer formed of a semiconductor material. The electrolyte includes at least one of pyrazole and a pyrazole derivative.

The dye may be a first metal complex which has a terpyridyl group and not more than one thiocyanate group. Alternatively, the dye may be a second metal complex which has two or more bipyridyl groups, and does not have a thiocyanate group.

The pyrazole derivative may be obtained by substituting one or two of hydrogen atoms constituting the pyrazole with an atom other than the hydrogen atom or an atom group, or may be obtained by substituting the hydrogen atom constituting the pyrazole with at least one of a methyl group, an ethyl group, a propyl group, and a butyl group.

### Advantageous Effects of Invention

In the photoelectric conversion element according to the present invention, a decrease in performance due to heat is suppressed.

### Brief Description of Drawings

[Fig. 1] Fig. 1 is a cross-sectional view illustrating an example of a configuration of a photoelectric conversion element of the present invention.
[Fig. 2] Fig. 2 is a graph illustrating a result of an example.
[Fig. 3] Fig. 3 is a graph illustrating a result of a comparative example.
[Fig. 4] Fig. 4 is a graph illustrating a result of an example.
[Fig. 5] Fig. 5 is a graph illustrating a result of a comparative example.

## Description of Embodiments

Hereinafter, a photoelectric conversion element of the present invention will be described with reference to the drawings. Note that in the drawings of the present invention, the same reference numerals indicate the same parts or the corresponding parts. In addition, dimensional relationships such as a length, a width, a thickness, and a depth are suitably changed for the sake of clarification and simplification of the drawings, and do not indicate actual dimensional relationships.

### [Photoelectric Conversion Element]

Fig. 1 is a cross-sectional view illustrating an example of a configuration of a photoelectric conversion element according to the present invention. In a photoelectric conversion element 10 illustrated in Fig. 1, a conductive layer 2, a photoelectric conversion layer 3, and a counter electrode 8 are sequentially disposed on a support substrate 1, and a charge transport layer 4 is formed by filling a space between the photoelectric conversion layer 3 and the counter electrode 8 with an electrolyte. It is preferable that the photoelectric conversion layer 3 and the charge transport layer 4 are sealed with a sealing portion 9.

In the photoelectric conversion layer 3, at least one of a dye formed of a compound having not more than one

thiocyanate group and a dye formed of a compound not having a thiocyanate group is adsorbed onto a porous semiconductor layer formed of a semiconductor material. In addition, the electrolyte includes at least one of pyrazole and a pyrazole derivative.

As examples of a factor of degradation of performance of the photoelectric conversion element by heat, degradation of a dye or the like due to heat is included separately from a leakage of the electrolyte. However, in the photoelectric conversion element 10 illustrated in Fig. 1, at least one of a dye formed of a compound having not more than one thiocyanate group and a dye formed of a compound not having a thiocyanate group is used, and at least one of pyrazole and a pyrazole derivative is added to the electrolyte. Accordingly, it is considered that an interaction (an attractive force based on an electrostatic interaction) between a lone pair of nitrogen atoms in the pyrazole or the pyrazole derivative and the dye is induced, and thus degradation of the dye or the like due to heat is prevented. Therefore, heat resistance of the photoelectric conversion element 10 is significantly improved. For example, a decrease in photoelectric conversion efficiency of the photoelectric conversion element 10 due to heat is suppressed.

The thiocyanate group is negatively charged. Accordingly, a repulsive force based on an electrostatic interaction is generated between a lone pair of the nitrogen atoms in the pyrazole or the pyrazole derivative and the thiocyanate group. When the dye has two or more thiocyanate groups, an interaction exerting between the lone pair of the nitrogen atoms in the pyrazole or the pyrazole derivative and the dye may be weakened by the repulsive force described above, and thus degradation of the dye or the like due to heat may be caused. However, in the photoelectric conversion element 10 illustrated in Fig. 1, at least one of a dye formed of a compound having not more than one thiocyanate group and a dye formed of a compound not having a thiocyanate group is used, and thus the interaction exerting between a lone pair of the nitrogen atoms in the pyrazole or the pyrazole derivative and the dye is not weakened by the repulsive force described above, and thus an effect of preventing degradation of the dye due to heat can be effectively obtained.

In general, when titanium oxide is used as a semiconductor material forming a porous semiconductor layer and at least one of iodide ions and triiodide ions is included as redox species included in the electrolyte, photoelectric conversion efficiency of the photoelectric conversion element increases, but heat resistance thereof significantly decreases. However, in the photoelectric conversion element illustrated in Fig. 1, at least one of a dye formed of a compound having not more than one thiocyanate group and a dye formed of a compound not having a thiocyanate group is used and at least one of pyrazole and a pyrazole derivative is added to the electrolyte, and thus heat resistance thereof is significantly improved. Therefore, in the photoelectric conversion element illustrated in Fig. 1, when titanium oxide is used as the semiconductor material forming the porous semiconductor layer and at least one of the iodide ions and the triiodide ions is used as the redox species included in the electrolyte, not only a decrease in heat resistance is able to be prevented but also photoelectric conversion efficiency is able to be improved. Hereinafter, each constituent of the photoelectric conversion element 10 illustrated in Fig. 1 will be described.

<Support substrate>

A material forming the support substrate 1 is not particularly limited insofar as the material is able to be generally used in the support substrate of the photoelectric conversion element and can exhibit the effect of the present invention. However, light transmissivity is required for a portion which serves as a light-receiving surface of the photoelectric conversion element, and thus it is preferable that the support substrate 1 is formed of a material having light transmissivity. The support substrate 1, for example, may be a glass substrate formed of soda-lime glass, fused quartz glass, crystal quartz glass, or the like, or may be a flexible film formed of a heat resistance resin material. However, when the support substrate 1 is used as the light-receiving surface, the support substrate 1 may be desired to substantially transmit light of a wavelength having sensitivity which is effective for at least a dye described later (transmittance of the light described above, for example, is greater than or equal to 80%, and is preferably greater than or equal to 90%), and it is not necessary to have transmission properties with respect to light in the entire wavelength region.

As a material forming the flexible film (hereinafter, referred to as a "film"), for example, tetraacetylcellulose (TAC), polyethylene terephthalate (PET), polyphenylene sulfide (PPS), polycarbonate (PC), polyarylate (PA), polyether imide (PEI), a phenoxy resin, polytetrafluoroethylene, and the like are included.

When another layer is formed on the support substrate 1 with heating, for example, when a porous semiconductor layer is formed on the support substrate 1 with heating at approximately 250°C, it is particularly preferable to use polytetrafluoroethylene having heat resistance of higher than or equal to 250°C among the materials forming the film described above.

When a completed photoelectric conversion element 10 is attached to another structure, the support substrate 1 can be used. That is, it is possible to easily attach a peripheral portion of the support substrate 1 formed of a glass substrate or the like to the other supporting body by using a metal processed component and a screw.

The thickness of the support substrate 1 is not particularly limited, and in consideration of light transmissivity or the like, it is preferable that the thickness of the support substrate 1 is approximately 0.2 mm to 5 mm.

<Conductive Layer>

A material forming the conductive layer 2 is not particularly limited insofar as the material is able to be generally used in the conductive layer of the photoelectric conversion element and can exhibit the effect of the present invention. However, the conductive layer 2 serves as a light-receiving surface of the photoelectric conversion element 10, and thus light transmissivity is required, and therefore, it is preferable that the conductive layer 2 is formed of a material having light transmissivity. It is preferable that the conductive layer 2 is formed of, for example, indium tin complex oxide (ITO), tin oxide doped with fluorine (FTO), zinc oxide (ZnO), or the like. Similarly to the support substrate 1, the conductive layer 2 may be desired to substantially transmit light of a wavelength having sensitivity which is effective for at least a dye described later (transmittance of the light described above, for example, is greater than or equal to 80%, and is preferably greater than or equal to 90%), and it is not necessary to have transmission properties with respect to light in the entire wavelength region.

The thickness of the conductive layer 2 is not particularly limited, and it is preferable that the thickness of the conductive layer 2 is approximately 0.02 $\mu$m to 5 $\mu$m. It is preferable that film resistance of the conductive layer 2 is low as much as possible, and it is preferable that the film resistance of the conductive layer 2 is less than or equal to 40 $\Omega$/sq.

In the conductive layer 2, a metal lead wire may be disposed in order to reduce a resistance thereof. As a material of the metal lead wire, for example, platinum, gold, silver, copper, aluminum, nickel, titanium, or the like is included. The size of the metal lead wire is not particularly limited, but when the size of the metal lead wire excessively increases, a decrease in incident light intensity from the light-receiving surface may be caused. Accordingly, it is preferable that the size of the metal lead wire is approximately 0.1 mm to 4 mm.

In the present invention, the structure in which the conductive layer 2 is formed on a surface of the support substrate 1 is referred to as a "transparent electrode substrate 11". As such a transparent electrode substrate 11, for example, a transparent electrode substrate in which the conductive layer 2 of FTO is formed on the support substrate 1 of soda-lime float glass is included. The transparent electrode substrate is preferably used in the present invention.

<Photoelectric Conversion Layer>

The photoelectric conversion layer 3 includes a porous semiconductor layer formed of a semiconductor material. At least one of a dye formed of a compound having not more than one thiocyanate group and a dye formed of a compound not having a thiocyanate group is adsorbed onto the porous semiconductor layer, and the porous semiconductor layer is preferably filled with an electrolyte.

<Porous Semiconductor Layer>

As examples of the shape of the porous semiconductor layer, a bulk-like layer formed of a semiconductor material, a layer including a particle-like semiconductor material, a film formed of a semiconductor material in which a plurality of micropores is formed, and the like are included, and the film formed of a semiconductor material in which a plurality of micropores is formed is preferable. By using such film, an adsorbed amount of dye, a filling amount of electrolyte, and the like are able to be sufficiently ensured.

Porous properties of the porous semiconductor layer indicate that porosity is greater than or equal to 20% and a specific surface area is 0.5 $m^2/g$ to 300 $m^2/g$. When the specific surface area of the porous semiconductor layer is 0.5 $m^2/g$ to 300 $m^2/g$, it is possible to adsorb a large amount of dye, and thus it is possible to efficiently absorb solar light. From a viewpoint of sufficiently ensuring, for example, an adsorbed amount of dye, it is more preferable that the specific surface area of the porous semiconductor layer is approximately 10 $m^2/g$ to 200 $m^2/g$. In addition, when the porosity of the porous semiconductor layer is greater than or equal to 20%, it is possible to sufficiently diffuse electrolyte, and thus it is possible to smoothly return electrons to the photoelectric conversion layer 3. Here, the porosity of the porous semiconductor layer is obtained by calculating from the thickness of the porous semiconductor layer, the mass of the porous semiconductor layer, and the density of the semiconductor material. The specific surface area of the porous semiconductor layer is obtained by a BET method which is a gas adsorbing method.

The semiconductor material forming the porous semiconductor layer (hereinafter, simply referred to as a "semiconductor material") is not particularly limited insofar as the semiconductor material is generally used as a photoelectric conversion material. The semiconductor material may be, for example, a metal oxide such as titanium oxide, zinc oxide, tin oxide, iron oxide, niobium oxide, cerium oxide, tungsten oxide, nickel oxide, or strontium titanate, or may be cadmium sulfide, lead sulfide, zinc sulfide, indium phosphide, copper-indium sulfide ($CuInS_2$), $CuAlO_2$, $SrCu_2O_2$, or the like. As the material forming the porous semiconductor layer, one of the materials described above may be independently used, or a combination of two or more thereof may be used. From a viewpoint of photoelectric conversion efficiency, stability, and safety, it is preferable that titanium oxide is used as the material forming the porous semiconductor layer.

In the present invention, when titanium oxide is used as the material forming the porous semiconductor layer,

titanium oxide to be used may be narrowly-defined various titanium oxides such as anatase titanium oxide, rutile titanium oxide, amorphous titanium oxide, metatitanic acid, or orthotitanic acid, may be titanium hydroxide, or may be aqueous titanium oxide. These titanium oxides may be independently used, or may be used by being mixed. Either the anatase titanium oxide or the rutile titanium oxide is able to be obtained according to a manufacturing method or a heat history, but the anatase titanium oxide is typically obtained.

An average particle diameter of the semiconductor material is not particularly limited. However, when the average particle diameter of the semiconductor material is changed, it is possible to adjust light scattering properties of the photoelectric conversion layer 3, and thus it is preferable that the average particle diameter of the semiconductor material is suitably determined in consideration of this. Specifically, the light scattering properties depend on, for example, forming conditions of the photoelectric conversion layer 3, and thus it is not possible to describe in general, but when the average particle diameter of the semiconductor material increases, light scattering properties are improved. Therefore, when the porous semiconductor layer includes a semiconductor material having a large average particle diameter, the photoelectric conversion layer 3 including the porous semiconductor layer has excellent light scattering properties, and thus contributes to an improvement of a light capturing rate. In contrast, when the average particle diameter of the semiconductor material decreases, an adsorbing point of dye increases. Therefore, when the porous semiconductor layer includes a semiconductor material having a small average particle diameter, the amount of dye adsorbed onto the porous semiconductor layer increases. The porous semiconductor layer may be a single layer formed of a semiconductor material having an approximately identical average particle diameter, or may be formed by laminating a layer formed of a semiconductor material having a relatively small average particle diameter and a layer formed of a semiconductor material having a relatively large average particle diameter. In the semiconductor material having a relatively small average particle diameter, it is preferable that the average particle diameter is greater than or equal to 5 nm and less than 50 nm, and it is more preferable that the average particle diameter is greater than or equal to 10 nm and less than or equal to 30 nm. Accordingly, a sufficiently large effective surface area can be obtained with respect to a projected area, and thus an effect of enabling incident light to be converted into electric energy at a high yield is also able to be obtained. In the semiconductor material having a relatively large average particle diameter, it is preferable that the average particle diameter is greater than or equal to 50 nm, it is more preferable that the average particle diameter is greater than or equal to 50 nm and less than or equal to 600 nm, and it is further preferable that the average particle diameter is greater than or equal to 50 nm and less than or equal to 100 nm. Furthermore, from a viewpoint of effectively using incident light in photoelectric conversion, it is preferable that the average particle diameter of the semiconductor material is uniform to a certain degree, like a commercially available semiconductor material.

As described above, from a viewpoint of improving light scattering properties, it is preferable that the semiconductor material is titanium oxide having an average particle diameter of greater than or equal to 50 nm, and more preferable that the semiconductor material is titanium oxide having an average particle diameter of greater than or equal to 50 nm and less than or equal to 100 nm.

Herein, the average particle diameter is a value obtained by using a diffraction peak which is obtained by X-ray diffraction (XRD). Specifically, the average particle diameter can be obtained from a half width of a diffraction angle in $\theta/2\theta$ measurement of the XRD and a Scherrer equation. For example, when the semiconductor material is the anatase titanium oxide, a half width of a diffraction peak (in the vicinity of $2\theta = 25.3°$) corresponding to a (101) plane may be measured.

The thickness of the porous semiconductor layer is not particularly limited, and it is preferable that the thickness of the porous semiconductor layer is approximately 0.5 $\mu$m to 50 $\mu$m from a viewpoint of photoelectric conversion efficiency. In particular, when the average particle diameter of the semiconductor material is greater than or equal to 50 nm, the thickness of the porous semiconductor layer is preferably 0.1 $\mu$m to 40 $\mu$m, and is more preferably 5 $\mu$m to 20 $\mu$m. In addition, when the average particle diameter of the semiconductor material is greater than or equal to 5 nm and less than 50 nm, the thickness of the porous semiconductor layer is preferably 0.1 $\mu$m to 50 $\mu$m, and is more preferably 10 $\mu$m to 40 $\mu$m.

In the photoelectric conversion element provided with a commercially available glass plate (corresponding to the support substrate 1) with $SnO_2$ film (corresponding to the conductive layer 2) attached, an insulating layer is generally disposed between the photoelectric conversion layer formed of the porous semiconductor layer and the counter electrode. However, for example, as disclosed in Japanese Unexamined Patent Application Publication No. 2007-194039, a counter electrode or a conductive layer (corresponding to a second conductive layer 6 described later) forming the counter electrode may be formed on the photoelectric conversion layer including the porous semiconductor layer formed of the semiconductor material having a large average particle diameter (the average particle diameter is approximately 100 nm to 500 nm). However, when the average particle diameter of the semiconductor material forming a portion of the photoelectric conversion layer which is in contact with the counter electrode or the conductive layer forming the counter electrode is large, a decrease in mechanical strength of the photoelectric conversion layer is caused, and thus a problem may occur regarding the structure of the photoelectric conversion element. In such a case, the semiconductor material having a relatively small average particle diameter is blended to the semiconductor material having a relatively large

average particle diameter, and for example, the semiconductor material having a relatively small average particle diameter is blended at a ratio of less than or equal to 10 mass% of the entire semiconductor material, and thus mechanical strength of the photoelectric conversion layer may be strengthened.

<Dye>

Dye usable in the present invention functions as a photosensitizing dye, and is at least one of a dye formed of a compound having not more than one thiocyanate group and a dye formed of a compound not having a thiocyanate group.

It is preferable that the dye used in the present invention is a first metal complex which has a terpyridyl group and has not more than one thiocyanate group. When the dye used in the present invention is the first metal complex, it is considered that a portion other than the terpyridyl group of the first metal complex and a lone pair of nitrogen atoms in pyrazole or a pyrazole derivative included in the electrolyte cause an interaction (an attractive force based on an electrostatic interaction). In addition, it is considered that the first metal complex has not more than one thiocyanate group, and thus it is possible to prevent a decrease in the interaction described above due to the thiocyanate group. Accordingly, it is considered that a degradation of the first metal complex due to heat is suppressed, and thus heat resistance of the photoelectric conversion element 10 is significantly improved. For example, a decrease in photoelectric conversion efficiency of the photoelectric conversion element 10 due to heat is suppressed. Here, "the first metal complex has not more than one thiocyanate group" includes a case where the first metal complex does not have a thiocyanate group.

As an example of the first metal complex, a metal complex having one thiocyanate group which is described in Japanese Unexamined Patent Application Publication No. 2005-120042 or Japanese Unexamined Patent Application Publication No. 2005-162718 may be included. As the dye having one thiocyanate group which is described in Japanese Unexamined Patent Application Publication No. 2005-120042, for example, a metal complex which is represented by a general formula RuLL' (NCS) can be included. Here, L is a terpyridyl group, and may have at least one interlocking group such as a carboxyl group in the molecule, or each linking group may form a salt of alkali metal or quaternary ammonium ions. L' is represented by the following general formula (a). In the general formula (a), it is preferable that $R_1$ is $CF_3$, $R_2$ is H, and $R_3$ is CN.

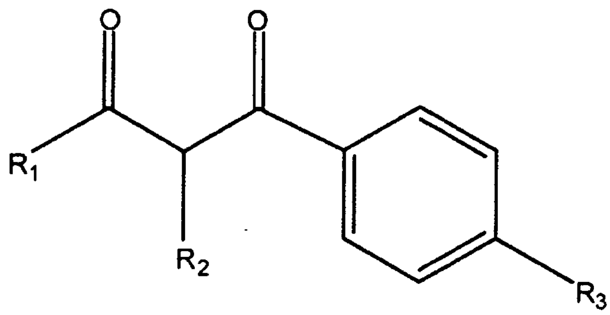

General Formula (a)

As the dye having one thiocyanate group which is described in Japanese Unexamined Patent Application Publication No. 2005-162718, for example, a metal complex represented by the following general formula (b) can be included. Here, at least one of $X_1$ to $X_3$ may be a linking group such as a carboxyl group or an ammonium carboxylate base, at least one thereof may be an alkyl group having 8 to 40 carbon atoms, and the remainder may be hydrogen. Y is a thiocyanate group.

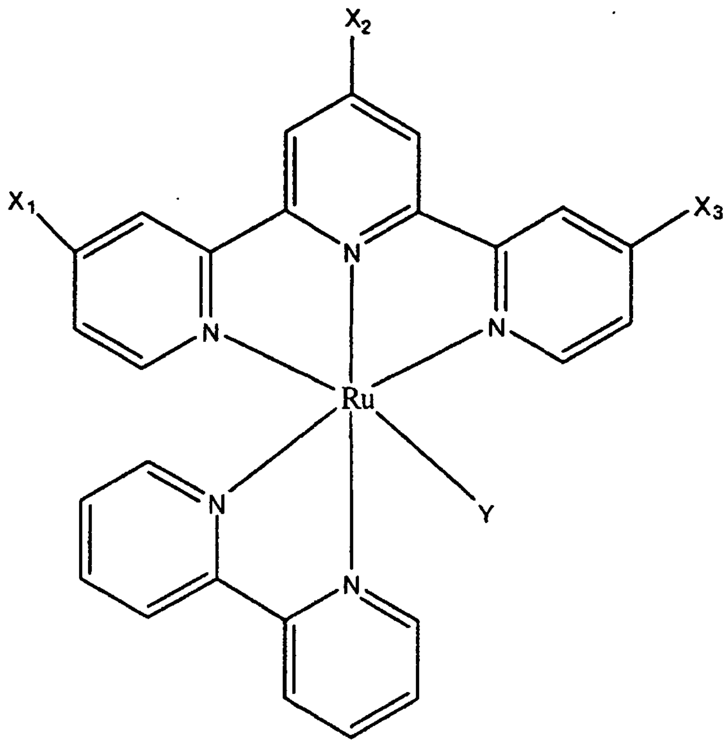

General Formula (b)

Dye usable in the present invention may be a second metal complex which has two or more bipyridyl groups and does not have a thiocyanate group. When the dye used in the present invention is the second metal complex, it is considered that a portion other than the bipyridyl group of the second metal complex and a lone pair of nitrogen atoms in pyrazole or a pyrazole derivative included in the electrolyte cause an interaction (an attractive force based on an electrostatic interaction). In addition, the second metal complex does not have a thiocyanate group, and thus it is possible to suppress a decrease in the interaction described above due to the thiocyanate group. Accordingly, it is considered that a degradation of the second metal complex due to heat is suppressed, and thus heat resistance of the photoelectric conversion element 10 is significantly improved. For example, a decrease in photoelectric conversion efficiency of the photoelectric conversion element 10 due to heat is suppressed.

As an example of the second metal complex, an organic dye which is represented by commercially available Ru470 (manufactured by Solaronix SA) or the like, a dye not having a thiocyanate group which is described in Japanese Unexamined Patent Application Publication No. 2005-162717, or the like can be included.

<<Ru470 Dye>>

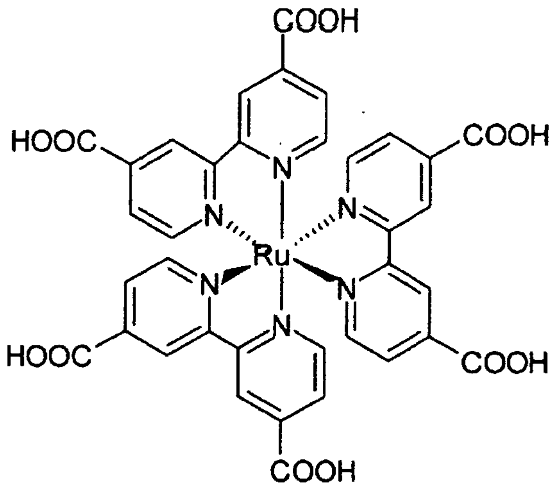

As the dye not having a thiocyanate group which is described in Japanese Unexamined Patent Application Publication No. 2005-162717, for example, a dye represented by the following general formula (c) can be included. Here, at least one of $X_4$ to $X_6$ may be a linking group such as a carboxyl group or an ammonium carboxylate base, and the remainder may be an alkyl group or hydrogen.

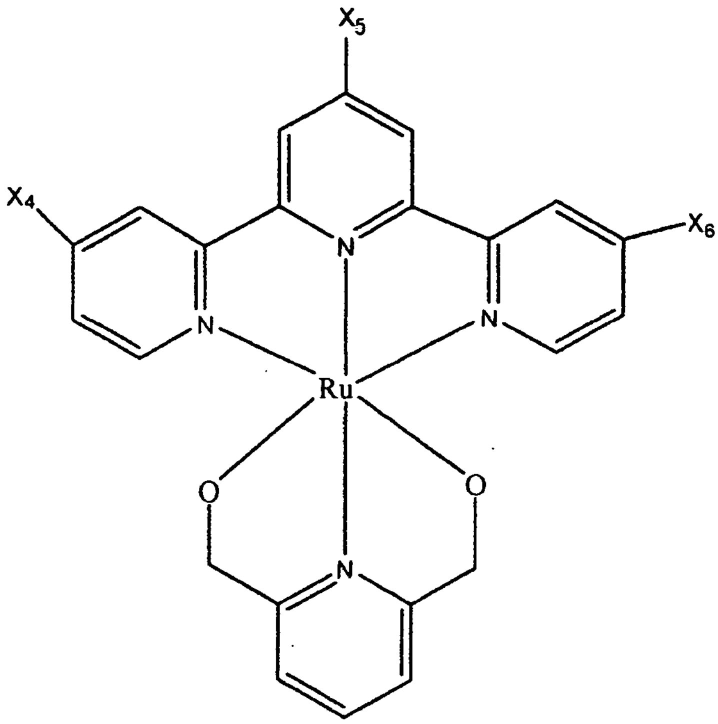

General Formula (c)

The dye used in the present invention may be independently the first metal complex, may be independently the second metal complex, or may be a mixture of the first metal complex and the second metal complex. When the dye used in the present invention is the mixture of the first metal complex and the second metal complex, a mixed ratio is not particularly limited, and it is preferable that the mixed ratio is (first metal complex):(second metal complex) = 1:9 to 9:1 (a mass ratio).

The dye used in the present invention is not limited to the metal complex, but may be an organic dye having not more than one thiocyanate group, may be an organic dye not having a thiocyanate group, or may be a mixture thereof. Examples of the organic dye include, for example, an azo dye, a quinine-based dye, a quinonimine-based dye, a quinacridone-based dye, a squarylium-based dye, a cyanine-based dye, a merocyanine-based dye, a triphenyl methane-based dye, a xanthene-based dye, a porphyrin-based dye, a perylene-based dye, an indigo dye, a phthalocyanine-based dye, a naphthalocyanine-based dye, or the like. As the organic dye not having a thiocyanate group, for example, an MK-II dye (manufactured by Soken Chemical & Engineering Co., Ltd.), a D131 dye (manufactured by Mitsubishi Chemical Corporation), or the like can be included. Note that an absorbance index of the organic dye is larger than an absorbance index of the dye formed of the metal complex.

<<MK-II Dye>>

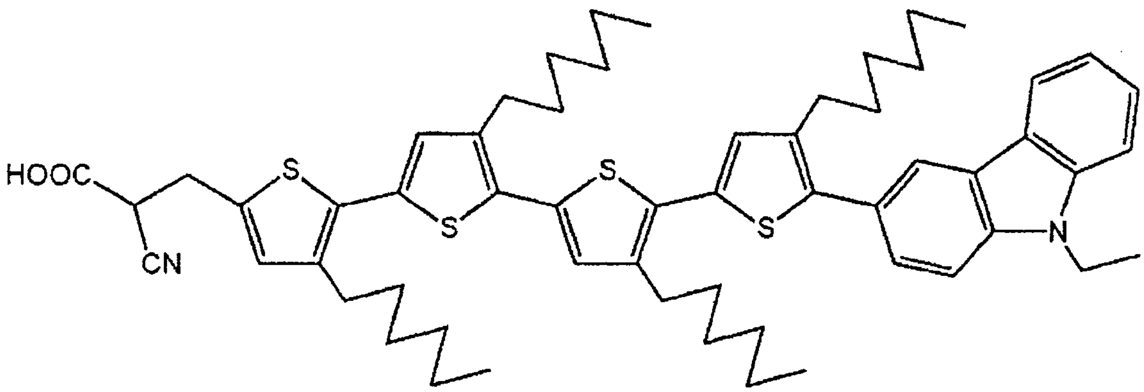

<<D131 Dye>>

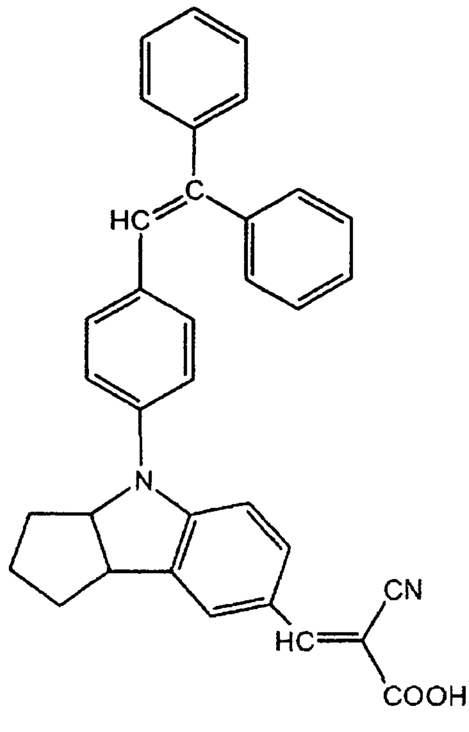

When the dye used in the present invention is the metal complex, center metal is not limited to ruthenium, and for example, may be Cu, Ni, Fe, Co, V, Sn, Si, Ti, Ge, Cr, Zn, Ru, Mg, Al, Pb, Mn, In, Mo, Y, Zr, Nb, Sb, La, W, Pt, Ta, Ir, Pd, Os, Ga, Tb, Eu, Rb, Bi, Se, As, Sc, Ag, Cd, Hf, Re, Au, Ac, Tc, Te, or the like.

The dye used in the present invention may be a phthalocyanine-based metal complex dye having not more than one thiocyanate group, may be a phthalocyanine-based metal complex dye not having a thiocyanate group, may be a ruthenium-based metal complex dye having one thiocyanate group, or may be a ruthenium-based metal complex dye not having a thiocyanate group. Among them, it is preferable that the dye used in the present invention is the

ruthenium-based metal complex dye having not more than one thiocyanate group or the ruthenium-based metal complex dye not having a thiocyanate group.

In order to solidly adsorb the dye onto the porous semiconductor layer, it is preferable that the dye has an interlocking group such as a carboxylic acid group, a carboxylic acid anhydride group, an alkoxy group, a hydroxyl group, a hydroxyalkyl group, a sulfonic acid group, an ester group, a mercapto group, or a phosphonyl group in the molecule, and it is more preferable that the dye has a carboxylic acid group or a carboxylic acid anhydride group. Here, the interlocking group provides electrical coupling which facilitates electron migration between an excited state of the dye and a conduction band of the semiconductor material.

An adsorbed amount of dye to the porous semiconductor layer is preferably greater than or equal to $1 \times 10^{-8}$ mol/cm$^2$ and less than or equal to $1 \times 10^{-6}$ mol/cm$^2$, and is more preferably greater than or equal to $5 \times 10^{-8}$ mol/cm$^2$ and less than or equal to $5 \times 10^{-7}$ mol/cm$^2$. When the adsorbed amount of the dye is less than $1 \times 10^{-8}$ mol/cm$^2$, a decrease in photoelectric conversion efficiency may be caused. In contrast, when the adsorbed amount of the dye exceeds $1 \times 10^{-6}$ mol/cm$^2$, a decrease in an open circuit voltage may be caused. However, when the adsorbed amount of the dye is greater than or equal to $1 \times 10^{-8}$ mol/cm$^2$ and less than or equal to $1 \times 10^{-6}$ mol/cm$^2$, it is possible to prevent a decrease in photoelectric conversion efficiency, and it is possible to prevent a decrease in an open circuit voltage. In addition, when two or more compounds are adsorbed onto the porous semiconductor layer as the dye, it is preferable that a total adsorbed amount of the dye satisfies the range described above. As a method for measuring the adsorbed amount of the dye to the porous semiconductor layer, the following method is included. After the dye is removed from the surface of the porous semiconductor by using an alkali solution, absorbance of the alkali solution is measured. By using a standard curve (the standard curve indicates a relationship between the concentration and the absorbance of the dye) which is prepared in advance, an amount of dye dissolved in the alkali solution is calculated from the measured absorbance, and thus the adsorbed amount of the dye is determined.

<Electrolyte>

The space between the photoelectric conversion layer 3 and the counter electrode 8 is filled with electrolyte. In other words, the charge transport layer 4 filled with the electrolyte exists between the photoelectric conversion layer 3 and the counter electrode 8. It is preferable that the porous semiconductor layer of the photoelectric conversion layer 3 is also filled with the electrolyte.

As the electrolyte, an electrolyte including a conductive polymer, redox species, or the like can be used. In order to efficiently inject electrons to the dye from the electrolyte, it is preferable that the highest unoccupied orbit level of the dye is lower than a Fermi level or an oxidation-reduction level thereof of the electrolyte (specifically, the redox species).

The electrolyte including the conductive polymer is not particularly limited insofar as the electrolyte is an electrolyte which is generally used in a cell, a solar cell, or the like. As the conductive polymer, for example, a hall transport material such as polyvinyl carbazole or triphenyl amine may be used, an electron transport material such as a fullerene derivative or tetranitrofluorenone may be used, a conductive polymer such as polypyrrole may be used, or an inorganic p type semiconductor such as copper iodide, copper thiocyanate, or nickel oxide may be used.

The electrolyte including the redox species is not particularly limited insofar as the electrolyte is an electrolyte which is generally used in a cell, a solar cell, or the like. Examples of the redox species include $I^-/I^{3-}$-based redox species, $Br^{2-}/Br^{3-}$-based redox species, $Fe^{2+}/Fe^{3+}$-based redox species, $Na_2S_x/Na_2S$-based redox species, $(SeCN)_2/SeCN^-$-based redox species, $(SCN)_2/SCN^-$-based redox species, $Co^{2+}/Co^{3+}$-based redox species, quinone/hydroquinone-based redox species, and the like.

As the $I^-/I^{3-}$-based redox species, for example, a combination of a metal iodide such as lithium iodide (LiI), iodide sodium (NaI), iodide potassium (KI), or iodide calcium ($CaI_2$) and iodine ($I_2$) may be used, or a combination of a tetraalkyl ammonium salt such as tetraethyl ammonium iodide (TEAI), tetrapropyl ammonium iodide (TPAI), tetrabutyl ammonium iodide (TBAI), or tetrahexyl ammonium iodide (THAI) and iodine may be used. Preferably, at least one of iodide ions and triiodide ions is used.

As the $Br^{2-}/Br^{3-}$-based redox species, for example, a combination of a metal bromide such as lithium bromide (LiBr), sodium bromide (NaBr), potassium bromide (KBr), or calcium bromide ($CaBr_2$) and bromine can be used. As the $Fe^{2+}/Fe^{3+}$-based redox species, for example, a combination of iron chloride (II) and iron chloride (III) may be used, or a combination of $K_3Fe(CN)_6$ and $K_4Fe(CN)_6$ may be used. As the $(SCN)_2/SCN^-$-based redox species, for example, a combination of $Pb(SCN)_2$ and NaSCN can be used.

As a solvent of the electrolyte, for example, a carbonate compound such as propylene carbonate, a nitrile compound such as acetonitrile, alcohols such as ethanol, water, an aprotic polar substance, or the like is included. Among them, it is particularly preferable to use the carbonate compound or the nitrile compound. One of these solvents can be independently used, or two or more thereof are able to be used by being mixed.

As necessary, an additive may be added to the electrolyte. As the additive, for example, a nitrogen-containing

aromatic compound such as t-butyl pyridine (TBP) may be used, or an imidazole salt such as dimethyl propyl imidazole iodide (DMPII), methyl propyl imidazole iodide (MPII), ethyl methyl imidazole iodide (EMII), ethyl imidazole iodide (EII), or hexyl methyl imidazole iodide (HMII) may be used.

The concentration of the redox species in the electrolyte, for example, is preferably 0.001 mol/L to 1.5 mol/L, and is more preferably 0.01 mol/L to 0.7 mol/L.

The electrolyte further includes at least one of the pyrazole and the pyrazole derivative. Accordingly, a lone pair of nitrogen atoms in the pyrazole or the pyrazole derivative and the dye used in the present invention induce an interaction, and thus a degradation of the dye due to heat or the like is prevented. As a result thereof, heat resistance of the photoelectric conversion element 10 is significantly improved, and for example, a decrease in photoelectric conversion efficiency due to heat is suppressed.

In the present invention, the pyrazole derivative indicates a pyrazole derivative in which at least one of the hydrogen atoms constituting the pyrazole is substituted with an atom other than the hydrogen atom or an arbitrary atom group (hereinafter, "the atom other than the hydrogen atom or the arbitrary atom group" is referred to as a "substituent"). Here, as the atom other than the hydrogen atom, for example, F, Cl, Br, I, or the like can be included. The atom group may be an alkyl group such as a methyl group, an ethyl group, a propyl group, or a butyl group, may be an atom group including these alkyl groups, or may be an amino group, a phenyl group, a thiophene group, a methoxy phenyl group, or the like other than the alkyl group. The pyrazole derivative may be a pyrazole derivative in which one or two of the hydrogen atoms constituting the pyrazole are substituted with a substituent, may be a pyrazole derivative in which the hydrogen atom constituting the pyrazole is substituted with at least one of a methyl group, an ethyl group, a propyl group, and a butyl group, or may be a pyrazole derivative in which a primary hydrogen atom of a pyrazole ring is not substituted. However, the present inventors have considered that the primary hydrogen atom of the pyrazole ring considerably affects the interaction with the dye. Accordingly, it is preferable that the pyrazole derivative is a pyrazole derivative in which the primary hydrogen atom of the pyrazole ring is not substituted.

Specific examples of the pyrazole derivative include, for example, 1-methyl pyrazole, 3-methyl pyrazole, 3,5-dimethyl pyrazole, 3,5-diisopropyl pyrazole, 3-amino-5-methyl pyrazole, and the like.

It is preferable that the electrolyte includes at least one of the pyrazole and the pyrazole derivative of greater than or equal to 0.01 mol/L and less than or equal to 10 mol/L, and it is more preferable that the electrolyte includes at least one of the pyrazole and the pyrazole derivative of greater than or equal to 0.05 mol/L and less than or equal to 5 mol/L. When the content of at least one of the pyrazole and the pyrazole derivative in the electrolyte is less than 0.01 mol/L%, it is difficult for the dye, and the pyrazole or the like to induce an interaction, and thus a degradation of the dye or the like due to heat may be caused. In contrast, when the content exceeds 10 mol/L, a problem that the pyrazole or the pyrazole derivative is hardly dissolved in the solvent may be caused. Furthermore, when the electrolyte includes the pyrazole and one or more pyrazole derivatives, it is preferable that a total content of the pyrazole and the one or more pyrazole derivatives in the electrolyte satisfies the range described above. The same applies to a case where the electrolyte does not include the pyrazole but includes two or more pyrazole derivatives.

<Counter electrode>

In the counter electrode 8, it is preferable that the second conductive layer 6 and a catalyst layer 5 are sequentially formed on the second support substrate 7, and it is preferable that the catalyst layer 5 faces the photoelectric conversion layer 3. It is preferable that a configuration of the second support substrate 7 is identical to that of the support substrate 1 described above, and it is preferable that a configuration of the second conductive layer 6 is identical to that of the conductive layer 2 described above. Furthermore, when the second conductive layer 6 also functions as the catalyst layer 5, for example, when the second conductive layer 6 also has an action of activating an oxidation-reduction reaction of the electrolyte, the counter electrode 8 may include either the catalyst layer 5 or the second conductive layer 6.

A material forming the catalyst layer 5 is not particularly limited insofar as the material is able to be generally used in the catalyst layer of the photoelectric conversion element. As the material forming the catalyst layer 5, for example, platinum, carbon black, Ketjen black, carbon nanotube, fullerene, or the like can be included.

The shape of the catalyst layer 5 is not particularly limited, and for example, a dense film-like layer, a porous film-like layer, a cluster-like layer, and the like are able to be included.

When the catalyst layer 5 made of platinum is formed, for example, the catalyst layer 5 can be formed on the second conductive layer 6 by a known method such as a sputtering method, or heat decomposition or electrodeposition of chloroplatinic acid. At this time, it is preferable that the thickness of the catalyst layer 5 is, for example, approximately 0.5 nm to 1000 nm.

When the catalyst layer 5 made of carbon such as carbon black, Ketjen black, carbon nanotube, or fullerene is formed, for example, it is preferable that carbon is dispersed in the solvent to be in the shape of a paste, and the paste is applied onto the second conductive layer 6 by a screen printing method or the like.

<Sealing Portion>

The sealing portion 9 has a function of retaining the transparent electrode substrate 11 and the counter electrode 8, a function of preventing a leakage of the charge transport layer 4, a function of receiving or absorbing a falling object or a stress (impact), and a function of absorbing deflection or the like acting on each of the transparent electrode substrate 11 and the counter electrode 8 when being used over a long period of time.

A material forming the sealing portion 9 is not particularly limited insofar as the material is able to be generally used in the sealing portion of the photoelectric conversion element and can exhibit the functions described above. As such a material, an ultraviolet curable resin, a thermosetting resin, or the like is included, and specifically, a silicone resin, an epoxy resin, a polyisobutylene-based resin, a hot-melt resin, a glass frit, or the like is included. The sealing portion 9 may be formed by independently using these materials, or the sealing portion 9 may be formed by laminating two or more layers of two or more of these materials.

When the sealing portion 9 formed of a silicone resin, an epoxy resin, or glass frit is formed, it is possible to form the sealing portion 9 by using a dispenser. When the sealing portion 9 formed of a hot-melt resin is formed, it is possible to form the sealing portion 9 by opening a patterned hole in a sheet-like hot-melt resin.

[Method of manufacturing Photoelectric Conversion Element]

A method of manufacturing the photoelectric conversion element illustrated in Fig. 1 will be described.

First, the transparent electrode substrate 11 is prepared in which the conductive layer 2 is formed on the support substrate 1. Specifically, a commercially available transparent electrode substrate may be prepared, or the conductive layer 2 may be formed on the support substrate 1 by a sputtering method, a thermal CVD method, or the like.

Next, the photoelectric conversion layer 3 is formed on the conductive layer 2. A method of forming the photoelectric conversion layer 3 is not particularly limited, and for example, the porous semiconductor layer can be formed by any method of the following (i) to (iv). Among the following (i) to (iv), it is preferable to use a screen printing method described in the following (i). Accordingly, the porous semiconductor layer which is comparatively thick tends to be manufactured at low cost.

(i) A paste including fine particles formed of a semiconductor material is applied onto the conductive layer 2 by a screen printing method, an ink jet method, or the like, and then is burned.
(ii) The porous semiconductor layer is formed on the conductive layer 2 by a CVD method, an MOCVD method, or the like using desired raw material gas.
(iii) The porous semiconductor layer is formed on the conductive layer 2 by a PVD method (for example, a vapor deposition method or a sputtering method) or the like using a solid raw material.
(iv) The porous semiconductor layer is formed on the conductive layer 2 by a sol-gel method, a method using an electrochemical oxidation-reduction reaction, or the like.

Next, a solution in which dye is dissolved (hereinafter, referred to as a "solution for adsorbing a dye") is prepared. As the dye, it is preferable to use at least one of the compounds described in <Dye> above. As a solvent of the solution for adsorbing a dye, for example, at least one of a carbonate compound such as propylene carbonate, a nitrile compound such as acetonitrile, alcohols such as ethanol, water, an aprotic polar substance, and the like can be used.

It is preferable that the concentration of the dye in the solution for adsorbing a dye is suitably adjusted according to the type of dye and solvent. In order to improve adsorbing function (adsorbing efficiency) of the dye to the porous semiconductor layer, it is preferable that the concentration of the dye in the solution for adsorbing a dye is high, and for example, it is preferable that the concentration is greater than or equal to $5 \times 10^{-4}$ mol/L. In order to reduce an interaction such as association between the dyes, an achromic hydrophobic compound such as a steroid compound having a carboxyl group may be adsorbed together.

Next, the transparent electrode substrate 11 in which the porous semiconductor layer is formed is immersed in the solution for adsorbing a dye. Accordingly, the dye in the solution for adsorbing a dye is adsorbed onto the porous semiconductor layer. At this time, it is preferable that immersion conditions are suitably adjusted.

Next, the second conductive layer 6 and the catalyst layer 5 are sequentially formed on the second support substrate 7. As a method of forming the second conductive layer 6, a method identical to the method of forming the conductive layer 2 can be used. As a method of forming the catalyst layer 5, the method described in <Counter electrode> above can be used.

Next, the electrolyte including at least one of pyrazole and a pyrazole derivative is prepared. Specifically, the electrolyte described in <Electrolyte> above may be prepared.

Next, the sealing portion 9 is arranged to surround the porous semiconductor layer onto which the dye is adsorbed. The transparent electrode substrate 11 and the counter electrode 8 are arranged such that the porous semiconductor layer and the catalyst layer 5 of the counter electrode 8 face each other, and then the transparent electrode substrate 11 is fixed to the counter electrode 8 by the sealing portion 9. After that, electrolyte is injected from the hole which is formed in the transparent electrode substrate 11 or the counter electrode 8 in advance to the inside of the region surrounded by the sealing portion 9. After that, when the hole is closed, the photoelectric conversion element illustrated in Fig. 1 is manufactured.

<Solar Cell Module>

In a solar cell module according to the present invention, photoelectric conversion elements according to the present invention (for example, the photoelectric conversion elements illustrated in Fig. 1) are connected in series. Accordingly, it is possible to provide a solar cell module in which a decrease in performance due to heat is suppressed and photoelectric conversion efficiency is improved. Examples

Hereinafter, the present invention will be more specifically described with reference to examples, but the present invention is not limited thereto. Furthermore, hereinafter, unless otherwise specifically noted, the thickness of each layer was measured by using a surface roughness and contour shape measuring instrument (manufactured by Tokyo Seimitsu Co., Ltd., a product name: Surfcom 1400A).

<Examples 1 to 7>

(Formation of Photoelectric Conversion Layer)

A commercially available titanium oxide paste (manufactured by Solaronix SA, a product name of Ti-Nanoxide D/SP, and an average particle diameter of 13 nm) was applied onto a glass plate (manufactured by Nippon Sheet Glass Co., Ltd., a $SnO_2$ film (a transparent conductive film) doped with fluorine was formed on the glass plate) by a doctor blade method. Next, the commercially available titanium oxide paste was preliminary dried at 300°C for 30 minutes, and then was burned at 500°C for 40 minutes. A series of these steps was performed twice, and thus a titanium oxide film (the porous semiconductor layer) having a thickness of 12 μm was obtained.

Subsequently, the glass plate on which the titanium oxide film is formed was immersed in the solution for adsorbing a dye at room temperature for 80 hours. The glass plate immersed in the solution for adsorbing a dye was cleaned by ethanol and was dried at approximately 60°C for approximately 5 minutes. Accordingly, the dye was adsorbed onto the titanium oxide film.

As the dye included in the solution for adsorbing a dye, an MK-II dye (manufactured by Soken Chemical & Engineering Co., Ltd.) was used in Example 1, a D131 dye (manufactured by Mitsubishi Chemical Corporation) was used in Example 2, a dye represented by the general formula (b) described above ($X_1$ is n-$C_{19}H_{39}$, $X_2$ is COOH, $X_3$ is n-$C_{19}H_{39}$, and Y is a thiocyanate group) was used in Example 3, a Ru470 dye (manufactured by Solaronix SA) was used in Example 4, a dye represented by the general formula (c) described above (all of $X_4$ to $X_6$ are COOH) was used in Example 5, a dye represented by the following chemical formula (I) was used in Example 6, and a dye represented by the following chemical formula (II) was used in Example 7. In each of the examples, the dye was dissolved in ethanol such that the concentration of the dye was $4 \times 10^{-4}$ mol/L.

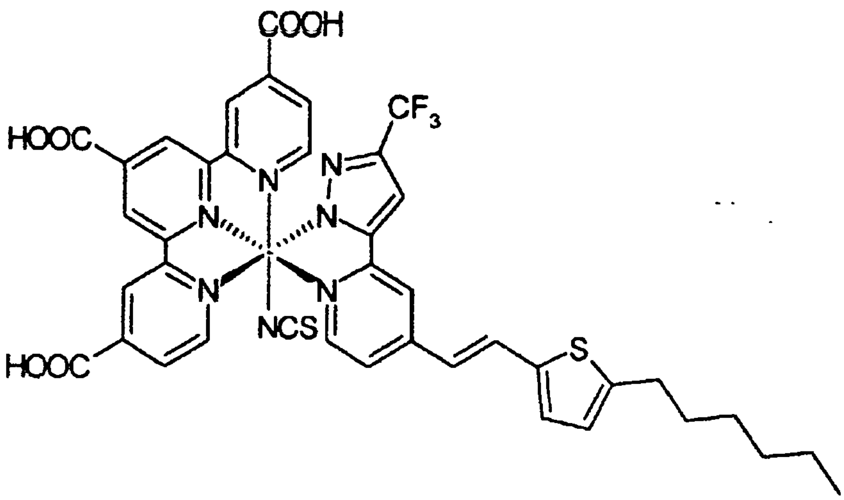

Chemical Formula (I)

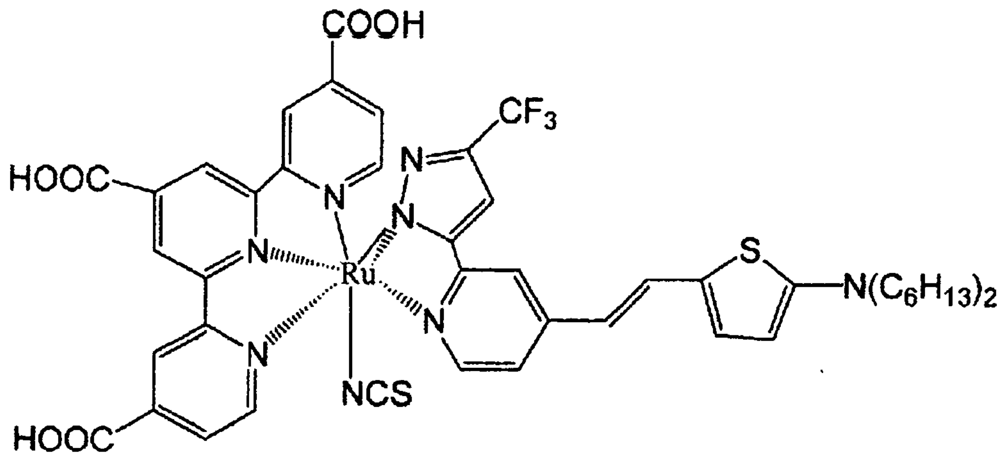

Chemical Formula (II)

A dye represented by the chemical formula (I) described above was obtained by a method described in the following scheme.

(7-1) Preparation of Compound d-1-2

25 g of a compound d-1-1 (2-acetyl-4-methyl pyridine) was dissolved in 200 ml of THF (tetrahydrofuran), 18.9 g of sodium ethoxide was added while being stirred at 0°C in a nitrogen atmosphere, and was stirred for 15 minutes. After that, 28.9 g of ethyl trifluoroacetate was dropped and was stirred at 70°C for 20 hours in the outside. After the temperature returned to the room temperature, an aqueous ammonium chloride solution was dropped and separated, an organic layer was condensed, and thus a roughly purified product d-1-2 (72.6 g) was obtained.

(7-2) Preparation of Compound d-1-3

72.6g of a compound d-1-2 was dissolved in 220 ml of ethanol, and 5.6 ml of hydrazine monohydrate was added while being stirred at room temperature in a nitrogen atmosphere, and was heated at 90°C for 12 hours in the outside. After that, 5 ml of a concentrated hydrochloric acid was added, and was stirred for 1 hour. After condensation, extraction and separation were performed by 150 ml of sodium bicarbonate water and 150 ml of ethyl acetate, and then an organic layer was condensed. The organic layer was crystallized again by acetonitrile, and thus a compound d-1-3 (31.5 g) was obtained.

(7-3) Preparation of Compound d-1-5

23.1 ml of an n-butyl lithium hexane solution of 1.6 M was dropped while stirring 4.1 g of diisopropyl amine and 30 ml of tetrahydrofuran at -40°C in a nitrogen atmosphere, and then was stirred for 2 hours. After that, the compound d-1-3 (4.0 g) was added and was stirred at 0°C for 80 minutes, and then a solution in which a compound d-1-4 (3.45 g) was dissolved in 15 ml of tetrahydrofuran was dropped. After that, the mixture was stirred at 0°C for 80 minutes, and was stirred at room temperature for 5 hours. After that, a chloride ammonium solution was added, and extraction and separation were performed by ethyl acetate. An organic layer was condensed and was purified by a silica gel column chromatography, and then a compound d-1-5 (5.7 g) was obtained.

(7-4) Preparation of Compound d-1-6

The compound d-1-5 (5.0 g) and 5.9 g of pyridinium paratoluene sulfonic acid (PPTS) were added to 50 ml of toluene, and heating and refluxing was performed for 5 hours in a nitrogen atmosphere. After condensation, separation was performed by saturated sodium bicarbonate water and methylene chloride, and an organic layer was condensed. The obtained crystal was crystallized again by methanol and methylene chloride, and thus a compound d-1-6 (4.3 g) was obtained.

The structure of the obtained compound d-1-6 was confirmed by mass spectrum (MS) measurement.
MS-ESI m/z = 404.2$(M-H)^+$

(7-5) Preparation of Compound d-1-9

A compound d-1-7 (1.22 g) and the compound d-1-6 (1.62 g) were added to 150 ml of N-methyl pyrrolidone (NMP), and were stirred at 70°C for 3 hours in a nitrogen atmosphere. After that, a compound d-1-8 (1.63 g) was added, and the mixture was heated and stirred at 160°C for 8 hours. After that, ammonium thiocyanate (10.7 g) was added, and the mixture was stirred at 160°C for 8 hours. After condensation, water was added and was filtered. After the filtered product was purified by the silica gel column chromatography, added to a mixed solvent of 30 ml of acetone and 40 ml of an aqueous sodium hydroxide solution of 1 N, and was stirred at 65°C for 24 hours in the outside. The temperature returned to room temperature, pH was adjusted to be 3 by hydrochloric acid, the precipitate was filtered, and thus a dye (a roughly purified product, 3.3 g) represented by the chemical formula (I) described above was obtained.

The dye was dissolved in a methanol solution together with tetrabutyl ammonium hydroxide (TBAOH), and was purified by a SephadexLH-20 column. Fractions of the main layer were collected and were condensed, and then a solution of trifluoromethane sulfonate of 0.1 M was added, pH was adjusted to be 3, and the precipitate was filtered. Accordingly, a dye (2.4 g) represented by the chemical formula (I) described above was obtained.

The structure of the obtained dye was confirmed by MS measurement.
MS-ESI m/z = 928.1(M-H)+

The obtained dye was prepared such that a dye concentration in 340 μmol/l of a tetrabutyl ammonium hydroxide methanol solvent was 17 μmol/l, spectral absorption measurement was performed, and maximum absorption wavelength was 521 nm.

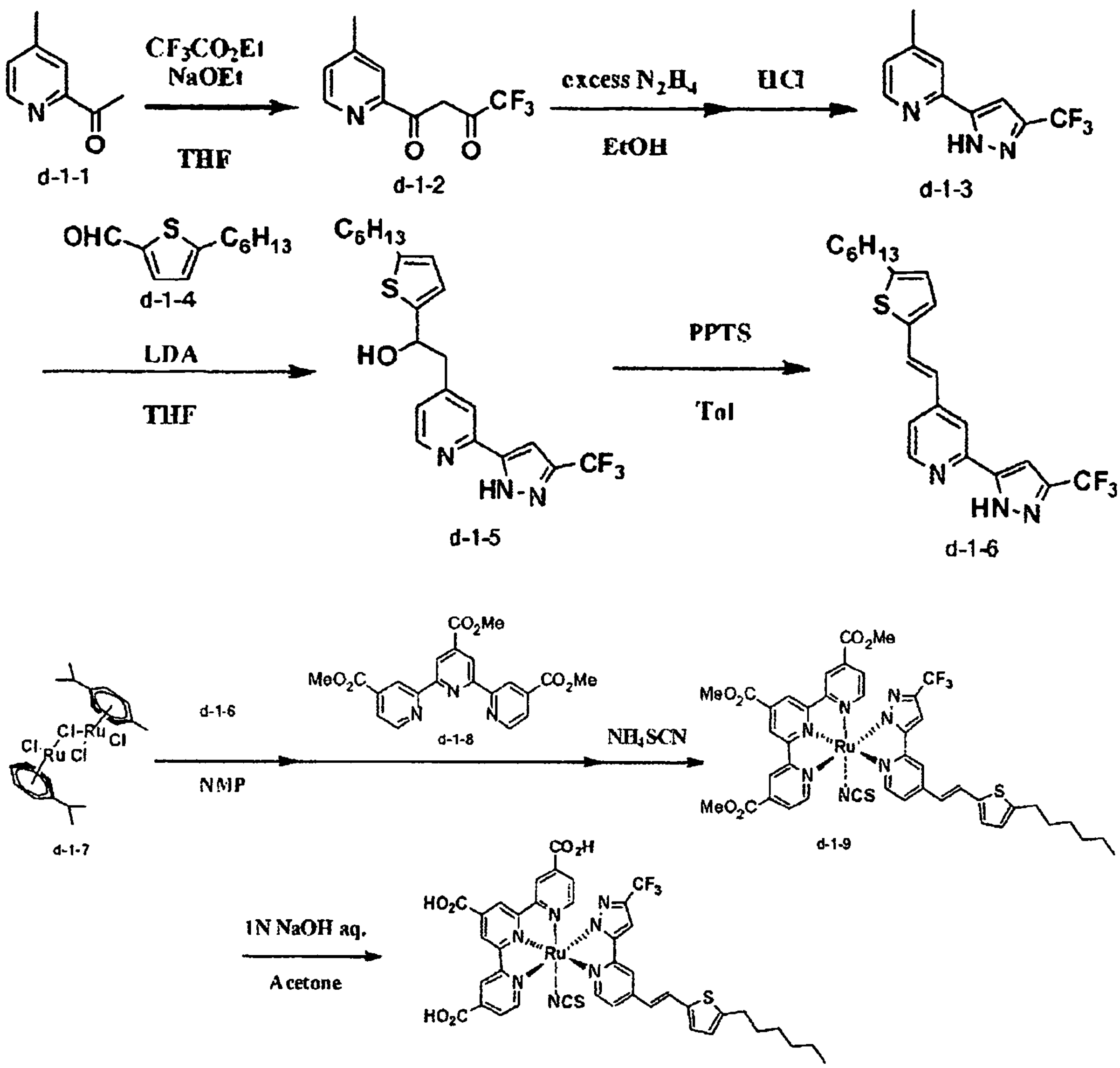

A dye represented by the chemical formula (II) described above was obtained by the method of preparing the dye represented by the chemical formula (I) described above except that the compound d-1-4 was changed to the following compound d-2-3. The compound d-2-3 was prepared by a method described in the following scheme.

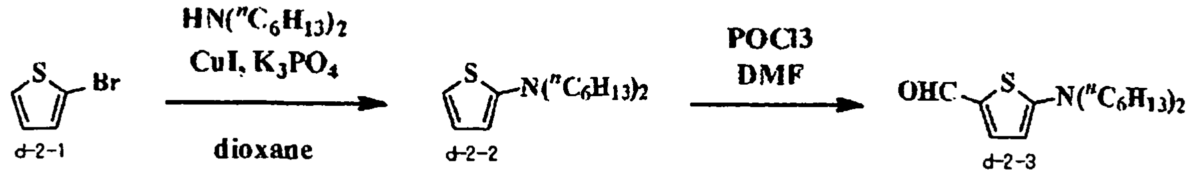

d-2-1 d-2-2 d-2-3

In Table 1 described below, the structure of the dye used in each example is shown.

[Table 1]

<table>
<tr><th></th><th>Dye</th><th>Number of NCS Groups[1)]</th><th>Number of Bipyridyl Groups[2)]</th><th>Number of Terpyridyl Groups[3)]</th><th>Additive to Electrolyte</th></tr>
<tr><td>Example 1</td><td>MK-II</td><td>0</td><td>0</td><td>0</td><td>3-Methyl Pyrazole</td></tr>
<tr><td>Example 2</td><td>D131</td><td>0</td><td>0</td><td>0</td><td>3-Methyl Pyrazole</td></tr>
<tr><td>Example 3</td><td>General Formula (b)</td><td>1</td><td>1</td><td>1</td><td>3-Methyl Pyrazole</td></tr>
<tr><td>Example 4</td><td>Ru470</td><td>0</td><td>3</td><td>0</td><td>3-Methyl Pyrazole</td></tr>
<tr><td>Example 5</td><td>General Formula (c)</td><td>0</td><td>0</td><td>1</td><td>3-Methyl Pyrazole</td></tr>
<tr><td>Example 6</td><td>Chemical Formula (I)</td><td>1</td><td>0</td><td>1</td><td>3-Methyl Pyrazole</td></tr>
<tr><td>Example 7</td><td>Chemical Formula (II)</td><td>1</td><td>0</td><td>1</td><td>3-Methyl Pyrazole</td></tr>
<tr><td>Example 8</td><td rowspan="6">Ru470</td><td rowspan="6">0</td><td rowspan="6">3</td><td rowspan="6">0</td><td>Pyrazole</td></tr>
<tr><td>Example 9</td><td>3,5-Dimethyl Pyrazole</td></tr>
<tr><td>Example 10</td><td>3-Amino-5-Methyl Pyrazole</td></tr>
<tr><td>Example 11</td><td>3,5-Diisopropyl Pyrazole</td></tr>
<tr><td>Example 12</td><td>1 ,3-Dimethyl Pyrazole</td></tr>
<tr><td>Example 13</td><td>1-Methyl Pyrazole</td></tr>
<tr><td>Example 14</td><td>Chemical Formula (I)</td><td>1</td><td>0</td><td>1</td><td>3-Methyl Pyrazole</td></tr>
<tr><td colspan="6">Number of NCS Groups[1)]: Number of Thiocyanate Groups Included in Dye<br>Number of Bipyridyl Groups[2)]: Number of Bipyridyl Groups Included in Dye<br>Number of Terpyridyl Groups[3)]: Number of Terpyridyl Groups Included in Dye</td></tr>
</table>

(Preparation of Electrolyte)

Iodine (manufactured by Sigma-Aldrich Corporation) was added to 3-methoxy propionitrile (manufactured by Sigma-Aldrich Corporation) such that the concentration was 0.15 mol/L, dimethyl propyl imidazole iodide (DMPII, manufactured by Shikoku Chemical Corporation) was added such that the concentration was 0.8 mol/L, guanidine thiocyanate (manufactured by Sigma-Aldrich Corporation) was added such that the concentration was 0.1 mol/L, and 3-methyl pyrazole (manufactured by Sigma-Aldrich Corporation) was added such that the concentration was 0.2 mol/L.

(Formation of Counter electrode)

One more glass plate (manufactured by Nippon Sheet Glass Co., Ltd., a $SnO_2$ film (the transparent conductive film) doped with fluorine is formed on the glass plate) was prepared separately from the glass plate used in (Formation of Photoelectric Conversion Layer). A platinum film was formed on the prepared glass plate (manufactured by Nippon Sheet Glass Co., Ltd.) at 0.1 Å/s by using a vapor deposition machine (a model name: ei-5, manufactured by ULVAC, Inc.). The thickness of the formed platinum film was 0.1 μm.

(Assembly)

The platinum film and the porous semiconductor layer were overlapped with each other by a spacer for preventing short circuit being interposed therebetween. The electrolyte was injected from a gap, and a side surface of a multi-layered body which was formed by overlapping the platinum film with the porous semiconductor layer was sealed with a resin (manufactured by ThreeBond Co., Ltd., "31X-101C"). Subsequently, a lead wire was attached to the $SnO_2$ film doped with fluorine which was formed on the glass plate. Accordingly, the photoelectric conversion element was obtained.

The photoelectric conversion element was irradiated with light (of an AM1.5 solar simulator) having an intensity of 1 kW/$m^2$, and photoelectric conversion efficiency was measured. After that, the photoelectric conversion element was retained in a thermostat bath at 85°C for 500 hours, change in photoelectric conversion efficiency over time was measured, and a retention rate of the photoelectric conversion efficiency was obtained. Furthermore, the retention rate of the photoelectric conversion efficiency was calculated by the following expression. It is indicated that a decrease in photoelectric conversion efficiency due to heat is suppressed as the retention rate of the photoelectric conversion efficiency is higher.

(Retention Rate of Photoelectric Conversion Efficiency) = (Photoelectric Conversion Efficiency after Photoelectric Conversion Element is Retained in Thermostat Bath at 85°C for 500 Hours) ÷ (Photoelectric Conversion Efficiency before Photoelectric Conversion Element is Retained in Thermostat Bath at 85°C).

The results are illustrated in Fig. 2. In Fig. 2, the results of the retention rate of photoelectric conversion efficiency with respect to a retention time in the thermostat bath at 85°C of Examples 1 to 7 are illustrated.

<Comparative Examples 1 and 2>

Each photoelectric conversion element of Comparative Example 1 and Comparative Example 2 was manufactured by the same method as that in Example 1 described above and in Example 4 described above except that the electrolyte was prepared without adding 3-methyl pyrazole. After that, the retention rate of the photoelectric conversion efficiency was obtained by the method described above. The results are illustrated in Fig. 3. In Fig. 3, the results of the retention rate of the photoelectric conversion efficiency with respect to the retention time in the thermostat bath at 85°C of Comparative Examples 1 to 8 are illustrated.

<Comparative Examples 3 and 4>

Photoelectric conversion elements of Comparative Example 3 and Comparative Example 4 were manufactured by the same method as that in Comparative Example 1 described above except that a Ru620-1H3TBA dye (manufactured by Solaronix SA) and a Ru535-bis-TBA dye (manufactured by Solaronix SA) were used as a dye, respectively. After that, the retention rate of the photoelectric conversion efficiency was obtained by the method described above. The results are illustrated in Fig. 3.

<<Ru620-1H3TBA Dye>>

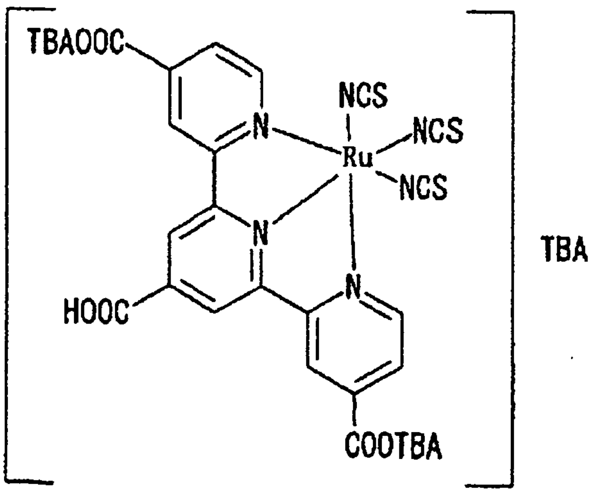

<<Ru535-bis-TBA Dye>>

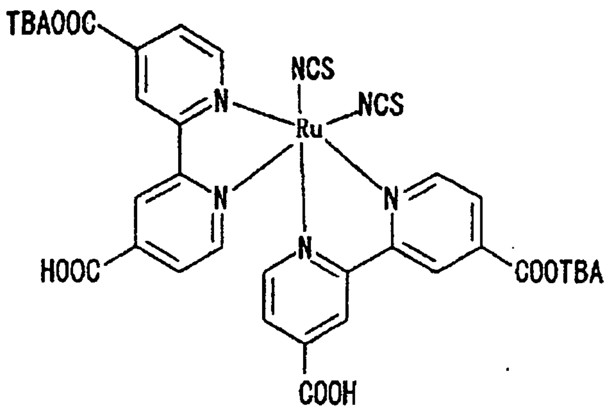

In the structure formula described above, "TBA" indicates tetrabutyl ammonium.

<Comparative Example 5>

The photoelectric conversion element of Comparative Example 5 was manufactured by the same method as that in Comparative Example 1 described above except that the dye represented by the chemical formula (I) described above was used. After that, the retention rate of the photoelectric conversion efficiency was obtained by using the method described above. The result is illustrated in Fig. 3.

<Comparative Examples 6 to 8>

Photoelectric conversion elements of Comparative Examples 6 to 8 were manufactured by the same method as that in Comparative Example 5 described above except that the electrolyte was prepared by adding N-methyl benzimidazole, t-butyl pyridine, and 1,3-dimethyl imidazole, respectively. After that, the retention rate of the photoelectric conversion efficiency was obtained by the method described above. The results are illustrated in Fig. 3.

In Table 2 described below, the structure of the dye used in each comparative example is shown.

[Table 2]

<table>
<tr><th></th><th>Dye</th><th>Number of NCS Groups 1)</th><th>Number of Bipyridyl Groups2)</th><th>Number of Terpyridyl Groups</th><th>Additive to Electrolyte</th></tr>
<tr><td>Comparative Example 1</td><td>MK-II</td><td>0</td><td>0</td><td>0</td><td>None</td></tr>
<tr><td>Comparative Example 2</td><td>Ru470</td><td>0</td><td>3</td><td>0</td><td>None</td></tr>
<tr><td>Comparative Example 3</td><td>Ru620-1H3TBA</td><td>3</td><td>0</td><td>1</td><td>None</td></tr>
<tr><td>Comparative Example 4</td><td>Ru535-bis-TBA</td><td>2</td><td>2</td><td>0</td><td>None</td></tr>
<tr><td>Comparative Example 5</td><td rowspan="4">Chemical Formula (I)</td><td rowspan="4">1</td><td rowspan="4">0</td><td rowspan="4">1</td><td>None</td></tr>
<tr><td>Comparative Example 6</td><td>N-Methyl Benzimidazole</td></tr>
<tr><td>Comparative Example 7</td><td>t-Butyl Pyridine</td></tr>
<tr><td>Comparative Example 8</td><td>1,3-Dimethyl Imidazole</td></tr>
<tr><td>Comparative Example 9</td><td>Ru620-1H3TBA</td><td>3</td><td>0</td><td>1</td><td>3-Methyl Pyrazole</td></tr>
<tr><td>Comparative Example 10</td><td>Ru535-bis-TBA</td><td>2</td><td>2</td><td>0</td><td>3-Methyl Pyrazole</td></tr>
<tr><td>Comparative Example 11</td><td>C101</td><td>2</td><td>2</td><td>0</td><td>3-Methyl Pyrazole</td></tr>
<tr><td>Comparative Example 12</td><td>CYC-B1</td><td>2</td><td>2</td><td>0</td><td>3-Methyl Pyrazole</td></tr>
<tr><td colspan="6">Number of NCS Groups1): Number of Thiocyanate Groups Included in Dye<br>Number of Bipyridyl Groups2): Number of Bipyridyl Groups Included in Dye<br>Number of Terpyridyl Groups3): Number of Terpyridyl Groups Included in Dye</td></tr>
</table>

As known from Fig. 2 and Fig. 3, the retention rate of the photoelectric conversion efficiency in Comparative Examples 1 to 8 was 0.5 to 0.8, but the retention rate of the photoelectric conversion efficiency in Examples 1 to 7 was greater than or equal to 0.9. As the reason therefor, the followings are considered. In Comparative Examples 1 to 5, the electrolyte does not include pyrazole and a pyrazole derivative. Accordingly, the dye did not cause an interaction with the additive to the electrolyte, and thus the heat resistance of the dye was decreased, and the retention rate of the photoelectric conversion efficiency was 0.5 to 0.8. In Comparative Examples 6 to 8, also, the dye did not cause the interaction with the additive to the electrolyte, and the heat resistance of the dye was decreased, and thus the retention rate of the photoelectric conversion efficiency was 0.5 to 0.8. In contrast, in Examples 1 to 7, the interaction between the dye and the pyrazole or the pyrazole derivative in the electrolyte was caused, the heat resistance of the dye was improved, and thus the retention rate of the photoelectric conversion efficiency indicated a high value of greater than or equal to 0.9.

<Examples 8 to 13>

Photoelectric conversion elements of Examples 8 to 13 were manufactured by the same method as in Example 4 described above except that pyrazole, 3,5-dimethyl pyrazole, 3-amino-5-methyl pyrazole, 3,5-diisopropyl pyrazole, 1,3-dimethyl pyrazole, and 1-methyl pyrazole were added to the electrolyte instead of 3-methyl pyrazole, respectively. In each example, the concentration of the pyrazole or the pyrazole derivative in the electrolyte was 0.2 mol/L. After that, the retention rate of the photoelectric conversion efficiency was obtained by the method described above. In Fig. 4, the results of the retention rate of the photoelectric conversion efficiency with respect to the retention time in the thermostat bath at 85°C of Examples 8 to 13 are illustrated.

<Comparative Examples 9 to 12>

Photoelectric conversion elements of Comparative Examples 9 to 12 were manufactured by the same method as that in Example 1 described above except that a Ru620-1H3TBA dye (manufactured by Solaronix SA), a Ru535-bis-TBA dye (manufactured by Solaronix SA), a C101 dye (manufactured by Solaronix SA), and a CYC-B1 dye (manufactured by Solaronix SA) were used as the dye, respectively. After that, the retention rate of the photoelectric conversion efficiency was obtained by the method described above. In Fig. 5, the results of the retention rate of the photoelectric conversion efficiency with respect to the retention time in the thermostat bath at 85°C of Comparative Examples 9 to 12 are illustrated.

<<C101 Dye>>

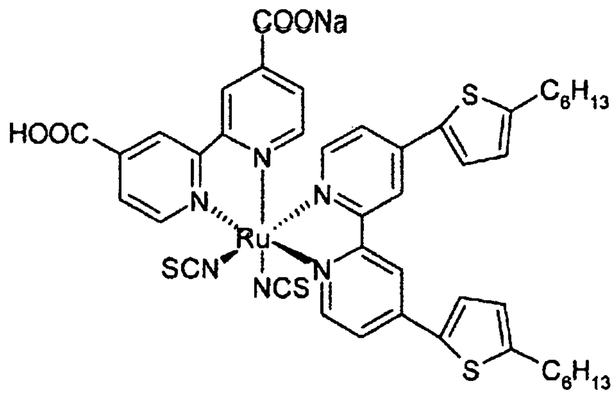

<<CYC-B1 Dye>>

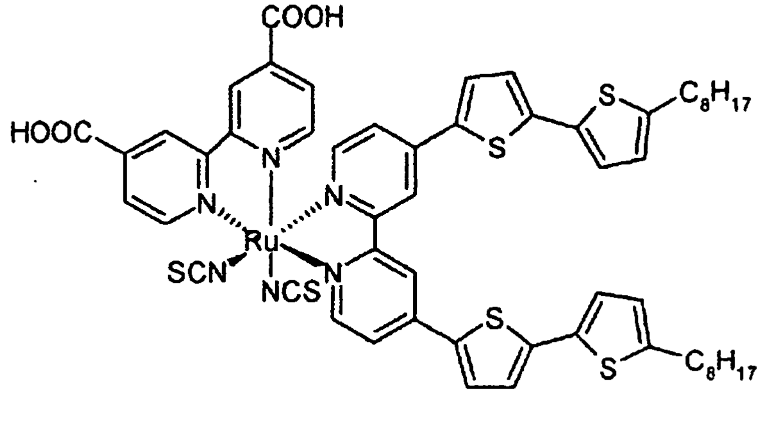

As known from Fig. 4, in Examples 8 to 13, the retention rate of the photoelectric conversion efficiency was greater than or equal to 0.9. Accordingly, even when the pyrazole or the pyrazole derivative which was used in Examples 8 to 13 was used instead of 3-methyl pyrazole, it was confirmed that the same effect as that of Example 1 was able to

be obtained.

As known from Fig. 5, in Comparative Examples 9 to 12, the retention rate of the photoelectric conversion efficiency was less than 0.9. As the reason therefor, the following is considered. In Comparative Examples 9 to 12, the electrolyte included the pyrazole derivative, but the dye was different from the dye used in the present invention. Accordingly, the dye did not cause an interaction with the additive to the electrolyte, and thus the heat resistance of the dye was decreased, and the retention rate of the photoelectric conversion efficiency was less than 0.9. In contrast, in Examples 8 to 13, the interaction between the dye and the pyrazole or the pyrazole derivative in the electrolyte was caused, the heat resistance of the dye was improved, and thus the retention rate of the photoelectric conversion efficiency indicated a high value of greater than or equal to 0.9.

<Example 14>

The photoelectric conversion module of Example 14 was manufactured by the following method.

A glass substrate of 70 mm × 70 mm × a thickness of 4 mm (manufactured by Nippon Sheet Glass Co., Ltd., a glass attached with $SnO_2$ film) was prepared. The prepared glass substrate was constituted by forming the conductive layer 2 formed of a $SnO_2$ film on the substrate 1 formed of glass.

The conductive layer 2 was irradiated with laser light (YAG laser, a fundamental wavelength: 1.06 μm, manufactured by Seishin Trading Co., Ltd.), a part of the conductive layer 2 was evaporated, and six linear scribes were formed.

A commercially available titanium oxide paste (manufactured by Solaronix SA, a product name of Ti-Nanoxide D/SP, and an average particle diameter of 13 nm) was applied onto the glass substrate by a doctor blade method. At this time, the titanium oxide paste described above was applied onto the conductive layer 2 on both side of a scribe line, and thus seven coated portions which were rectangular in plan view were formed on the glass substrate described above. Next, the coating was preliminary dried at 300°C for 30 minutes, and then was burned at 500°C for 40 minutes. These exemplary steps were performed twice, and thus a titanium oxide film (the porous semiconductor layer) having a thickness of 12 μm was obtained.

A zirconium oxide paste which was prepared in advance was printed on the titanium oxide film described above by using a printing machine LS-34TVA (manufactured by Newlong Seimitsu Kogyo Co., Ltd.). After that, the zirconium oxide paste was preliminary dried at 300°C for 30 minutes, and was burned at 500°C for 40 minutes. As a result, a zirconium oxide film (the porous insulating layer) having a thickness of 12 μm was obtained.

A platinum film was formed on the zirconium oxide film (the porous insulating layer) at 0.1 Å/s by using a vapor deposition machine (a model name: ei-5, manufactured by ULVAC, Inc.). The thickness of the formed platinum film was 0.1 μm. Next, a titanium film was formed on the platinum film at 5 Å/s by using the same vapor deposition machine. The thickness of the formed titanium film was 1000 nm.

A multi-layered body obtained as described above was immersed in the solution for adsorbing a dye used in Example 6 described above at room temperature for 80 hours. After that, the multi-layered body was cleaned by ethanol, and then was dried at approximately 60°C for approximately 5 minutes. As a result, the dye was adsorbed onto the porous semiconductor layer. The solution for adsorbing a dye was prepared by dissolving the dye in acetonitrile:t-butanol = 1:1 such that the concentration was $4 \times 10^{-4}$ mol/liter.

Cover glass was overlapped with the multi-layered body which was formed by adsorbing the dye onto the porous semiconductor layer. A side surface of the multi-layered body formed as described above was sealed with a resin 3035B (manufactured by ThreeBond Co., Ltd.). After that, the electrolytic solution of Example 1 described above was injected from a hole formed in the cover glass, a lead wire was attached to each electrode, and thus the photoelectric conversion module was obtained.

The obtained photoelectric conversion module was irradiated with light (of an AM1.5 solar simulator) having an intensity of 1 $kW/m^2$, and thus photoelectric conversion efficiency was measured. After that, the photoelectric conversion module was retained in a thermostat bath at 85°C for 500 hours, change in the photoelectric conversion efficiency over time was measured, and a retention rate of the photoelectric conversion efficiency was obtained. The obtained retention rate of the photoelectric conversion efficiency was 0.95, and in this example, the photoelectric conversion module having an excellent retention rate of the photoelectric conversion efficiency was obtained.

It should be considered that the embodiments and the examples disclosed herein are exemplification in all respects, and are not limitative. The scope of the present invention is not represented by the above description but by accompanying claims.

Reference Signs List

1 SUPPORT SUBSTRATE

2 CONDUCTIVE LAYER
3 PHOTOELECTRIC CONVERSION LAYER
4 CHARGE TRANSPORT LAYER
5 CATALYST LAYER
6 SECOND CONDUCTIVE LAYER
7 SECOND SUPPORT SUBSTRATE
8 COUNTER ELECTRODE
9 SEALING PORTION
10 PHOTOELECTRIC CONVERSION ELEMENT
11 TRANSPARENT ELECTRODE SUBSTRATE

## Claims

1. A photoelectric conversion element (10) comprising a conductive layer (2), a photoelectric conversion layer (3), and a counter electrode (8), at least the photoelectric conversion layer (3) being filled with an electrolyte,
wherein, in the photoelectric conversion layer (3), at least one of a dye formed of a compound having no more than one thiocyanate group and a dye formed of a compound not having a thiocyanate group is adsorbed onto a porous semiconductor layer formed of a semiconductor material, and
the electrolyte includes at least one of pyrazole and a pyrazole derivative.

2. The photoelectric conversion element according to Claim 1,
wherein the dye is a first metal complex which has a terpyridyl group and not more than one thiocyanate group.

3. The photoelectric conversion element according to Claim 1,
wherein the dye is a second metal complex which has two or more bipyridyl groups, and does not have a thiocyanate group.

4. The photoelectric conversion element according to any one of Claims 1 to 3,
wherein the pyrazole derivative is obtained by substituting one or two of hydrogen atoms constituting the pyrazole with an atom other than the hydrogen atom or an atom group.

5. The photoelectric conversion element according to any one of Claims 1 to 4,
wherein the pyrazole derivative is obtained by substituting a hydrogen atom constituting the pyrazole with at least one of a methyl group, an ethyl group, a propyl group, and a butyl group.

## Patentansprüche

1. Fotoelektrisches Umwandlungselement (10), aufweisend:

   eine leitfähige Schicht (2),
   eine fotoelektrische Umwandlungsschicht (3) und
   eine Gegenelektrode (8),
   wobei zumindest die fotoelektrische Umwandlungsschicht (3) mit einem Elektrolyten gefüllt ist,
   wobei in der fotoelektrischen Umwandlungsschicht (3) ein Farbstoff, der aus einer Verbindung besteht, die nicht mehr als eine Thiocyanatgruppe aufweist, und/oder ein Farbstoff, der aus einer Verbindung besteht, die keine Thiocyanatgruppe aufweist, auf eine aus einem Halbleitermaterial bestehende poröse Halbleiterschicht adsorbiert wird und
   der Elektrolyt Pyrazol und/oder ein Pyrazol-Derivat enthält.

2. Fotoelektrisches Umwandlungselement nach Anspruch 1,
wobei der Farbstoff ein erster Metallkomplex ist, der eine Terpyridylgruppe und nicht mehr als eine Thiocyanatgruppe aufweist.

3. Fotoelektrisches Umwandlungselement nach Anspruch 1,
wobei der Farbstoff ein zweiter Metallkomplex ist, der zwei oder mehr Bipyridylgruppen aufweist und keine Thiocyanatgruppe aufweist.

**4.** Fotoelektrisches Umwandlungselement nach einem der Ansprüche 1 bis 3,
wobei das Pyrazol-Derivat erhalten wird, indem ein oder zwei der Wasserstoffatome, die das Pyrazol bilden, durch ein anderes Atom als das Wasserstoffatom oder eine Atomgruppe substituiert werden.

**5.** Fotoelektrisches Umwandlungselement nach einem der Ansprüche 1 bis 4,
wobei das Pyrazol-Derivat erhalten wird, indem ein Wasserstoffatom, das das Pyrazol bildet, durch eine Methylgruppe, ein Ethylgruppe, eine Propylgruppe und/oder eine Butylgruppe substituiert wird.

**Revendications**

**1.** Elément de conversion photoélectrique (10) comprenant une couche conductrice (2), une couche de conversion photoélectrique (3), et une contre-électrode (8), au moins la couche de conversion photoélectrique (3) étant chargée d'un électrolyte,
dans lequel, dans la couche de conversion photoélectrique (3), au moins l'un parmi un colorant formé d'un composé ayant au plus un groupe thiocyanate et un colorant formé d'un composé n'ayant pas un groupe thiocyanate est adsorbé sur une couche semi-conductrice poreuse formée d'un matériau semi-conducteur, et
l'électrolyte contient au moins l'un parmi le pyrazole et un dérivé de pyrazole.

**2.** Elément de conversion photoélectrique selon la revendication 1, dans lequel le colorant est un premier complexe métallique qui a un groupe terpyridyle et au plus un groupe thiocyanate.

**3.** Elément de conversion photoélectrique selon la revendication 1, dans lequel le colorant est un deuxième complexe métallique qui a deux ou plus de deux groupes bipyridyle, et qui n'a pas un groupe thiocyanate.

**4.** Elément de conversion photoélectrique selon l'une quelconque des revendications 1 à 3, dans lequel le dérivé de pyrazole est obtenu par remplacement d'un ou deux atomes d'hydrogène constituant le pyrazole par un atome autre que l'atome d'hydrogène ou un groupe d'atomes.

**5.** Elément de conversion photoélectrique selon l'une quelconque des revendications 1 à 4, dans lequel le dérivé de pyrazole est obtenu par remplacement d'un atome d'hydrogène constituant le pyrazole par au moins l'un parmi un groupe méthyle, un groupe éthyle, un groupe propyle, et un groupe butyle.

FIG. 1

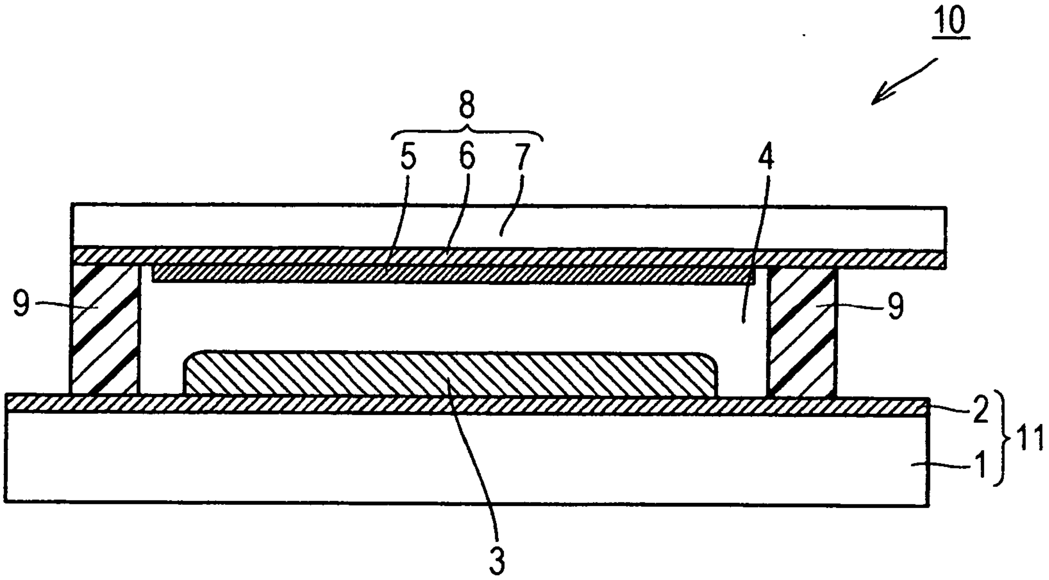
10
8
5 6 7
4
9
9
2
11
1
3

FIG. 2

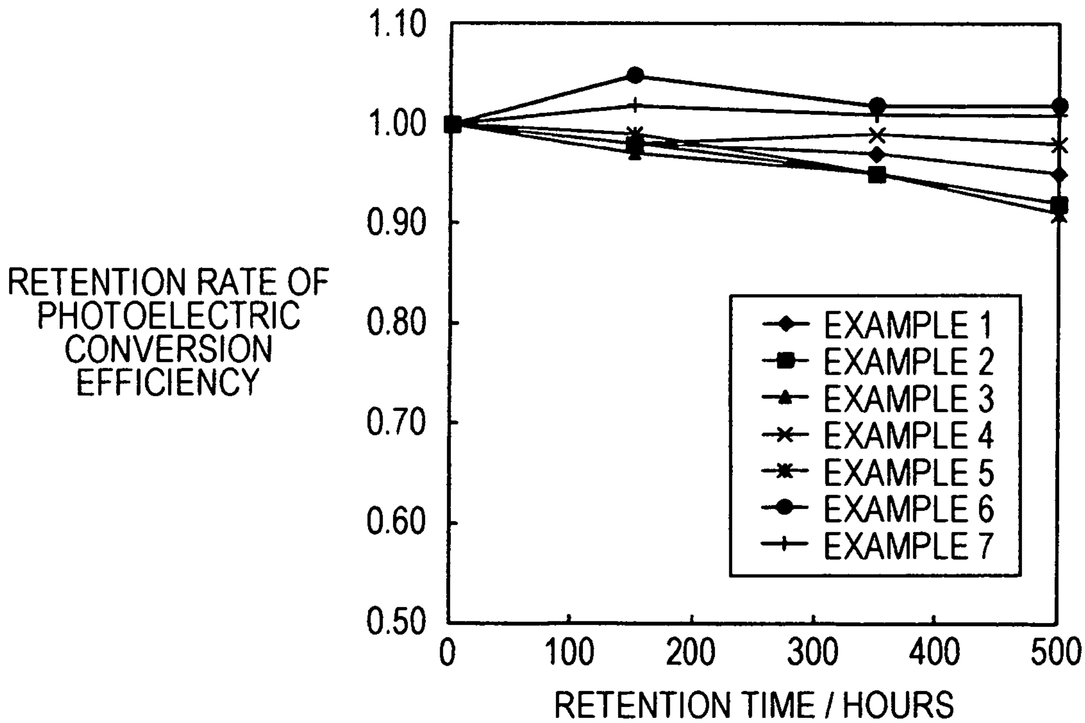
1.10
1.00
0.90
0.80
0.70
0.60
0.50
RETENTION RATE OF PHOTOELECTRIC CONVERSION EFFICIENCY
0
100
200
300
400
500
RETENTION TIME / HOURS
EXAMPLE 1
EXAMPLE 2
EXAMPLE 3
EXAMPLE 4
EXAMPLE 5
EXAMPLE 6
EXAMPLE 7

FIG. 3

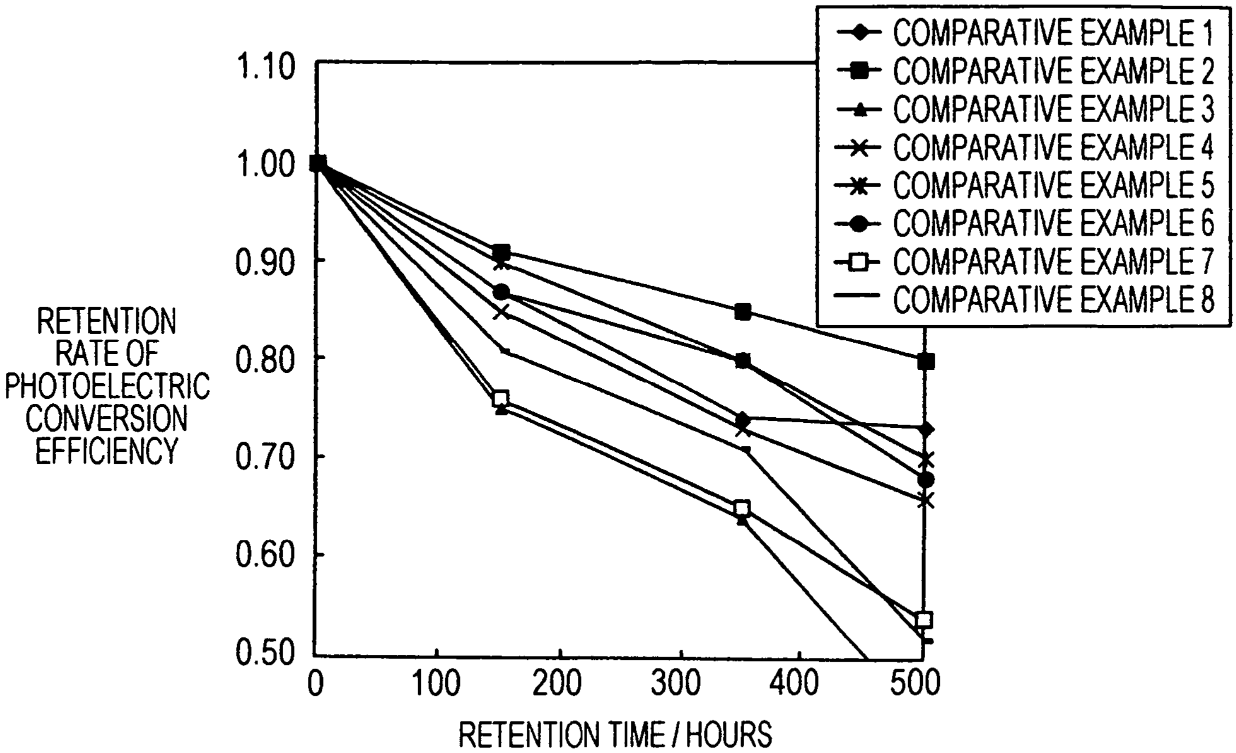
COMPARATIVE EXAMPLE 1
COMPARATIVE EXAMPLE 2
COMPARATIVE EXAMPLE 3
COMPARATIVE EXAMPLE 4
COMPARATIVE EXAMPLE 5
COMPARATIVE EXAMPLE 6
COMPARATIVE EXAMPLE 7
COMPARATIVE EXAMPLE 8
RETENTION RATE OF PHOTOELECTRIC CONVERSION EFFICIENCY
1.10
1.00
0.90
0.80
0.70
0.60
0.50
0
100
200
300
400
500
RETENTION TIME / HOURS

FIG. 4

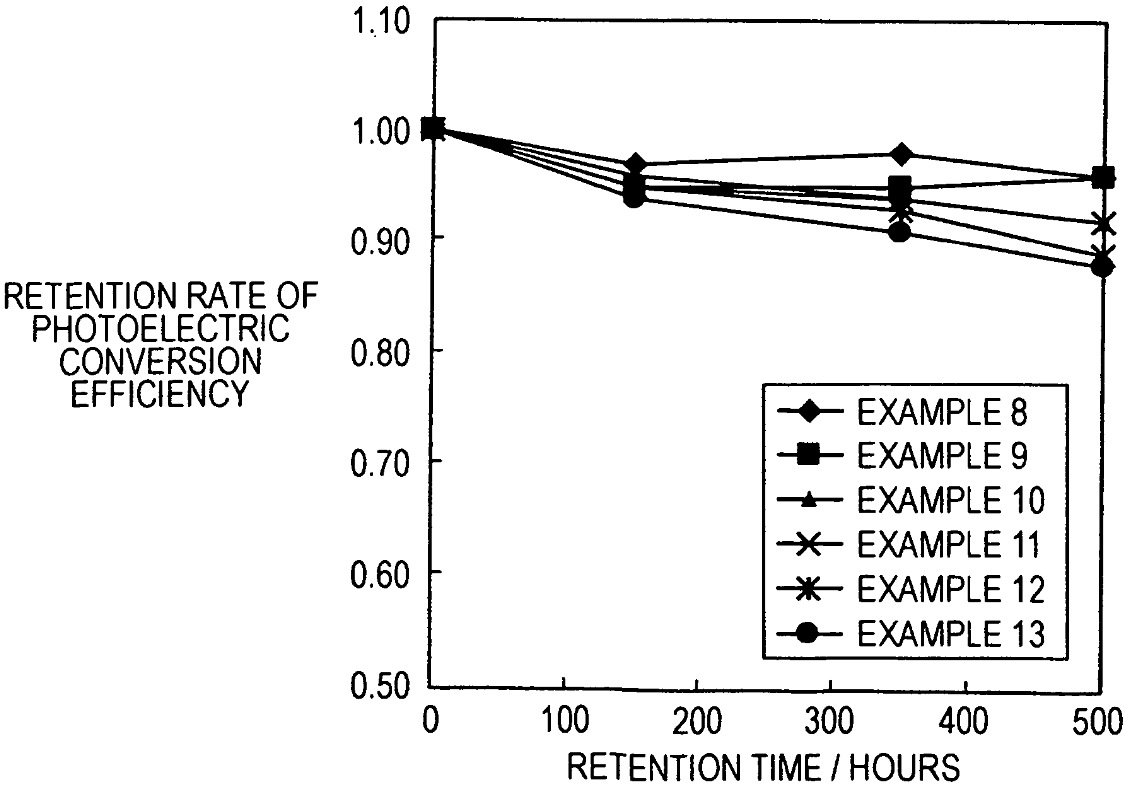
RETENTION RATE OF PHOTOELECTRIC CONVERSION EFFICIENCY
1.10
1.00
0.90
0.80
0.70
0.60
0.50
EXAMPLE 8
EXAMPLE 9
EXAMPLE 10
EXAMPLE 11
EXAMPLE 12
EXAMPLE 13
0
100
200
300
400
500
RETENTION TIME / HOURS

FIG. 5

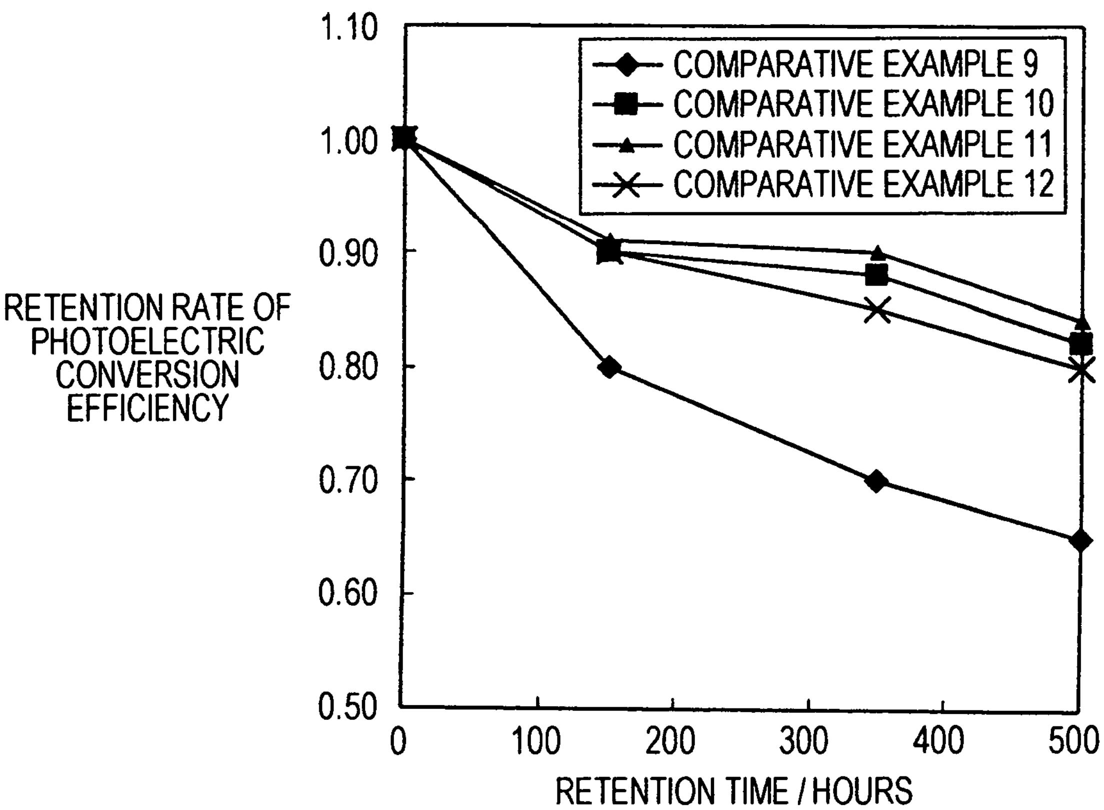
1.10
1.00
0.90
0.80
0.70
0.60
0.50
RETENTION RATE OF PHOTOELECTRIC CONVERSION EFFICIENCY
COMPARATIVE EXAMPLE 9
COMPARATIVE EXAMPLE 10
COMPARATIVE EXAMPLE 11
COMPARATIVE EXAMPLE 12
0
100
200
300
400
500
RETENTION TIME / HOURS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1220380 A
- JP 2003331936 A
- JP 2004047229 A
- JP 2005216490 A
- JP 2006134615 A
- US 2012073660 A1
- JP 2012243436 A
- JP 2007194039 A
- JP 2005120042 A
- JP 2005162718 A
- JP 2005162717 A


### Non-patent literature cited in the description


- **KUSAMA H. et al.** *Sol. Ener. Mater. and Sol. Cells,* 2004, vol. 85, 333-344
- **KUSAMA H. et al.** *J. Photochem. Photobiol. A,* 2004, vol. 169, 169-176